# EUROPÄISCHE PATENTSCHRIFT

(11) **EP 2 335 556 B1**
(45) Veröffentlichungstag und Bekanntmachung des Hinweises auf die Patenterteilung: **30.05.2018**
(21) Anmeldenummer: 10194669.7
(22) Anmeldetag: 13.12.2010
(51) Int. Cl.: A61B 1/00, A61B 1/04, G01N 21/27, A61B 5/00, A61B 5/1495

(54) **Prüfvorrichtung für ein optisches Untersuchungssystem**
Test device for an optical inspection system
Dispositif de contrôle d'un système d'analyse optique

(30) Priorität: 16.12.2009 DE 102009058660
(43) Veröffentlichungstag der Anmeldung: 22.06.2011
(73) Patentinhaber: Karl Storz SE & Co. KG, 78532 Tuttlingen (DE)
(72) Erfinder: Beck, Gerd, 78532, Tuttlingen (DE); Ehrhardt, André, 78532, Tuttlingen (DE); Glöggler, Bernhard, 78532, Tuttlingen (DE)

(56) Entgegenhaltungen:
- EP-A1- 1 728 464
- WO-A2-97/11636
- DE-A1- 19 638 809
- DE-A1- 19 855 853
- US-A1- 2003 078 477
- US-A1- 2003 105 195

## Beschreibung

Die vorliegende Erfindung bezieht sich auf eine Prüfvorrichtung für ein optisches Untersuchungssystem mit einer Lichtquelle und einer bildgebenden Einrichtung zur optischen Untersuchung eines Objekts innerhalb und außerhalb der Medizin. Die vorliegende Erfindung bezieht sich insbesondere auf eine Prüfvorrichtung für ein Endoskopiesystem mit einer Lichtquelle und einem Endoskop.

Zur Endoskopie in medizinischen oder nicht-medizinischen Anwendungen - im zweiten Fall auch als Boroskopie bezeichnet - werden Endoskopiesysteme aus jeweils einem Endoskop und einer Lichtquelle verwendet. Die Lichtquelle kann in das Endoskop, insbesondere in dessen distales Ende, integriert sein oder als separate Einheit bereitstehen, die über ein Lichtleitkabel mit dem Endoskop optisch gekoppelt ist. Licht der Lichtquelle tritt am distalen Ende des Endoskops aus und beleuchtet dort ein zu betrachtendes Objekt. Vom Objekt remittiertes Licht wird von einer Optik am distalen Ende des Endoskops aufgefangen und auf einen lichtempfindlichen Bildsensor geleitet oder, beispielsweise mittels eines geordneten Bündels von Lichtwellenleitern oder einer Stablinsenoptik, zum proximalen Ende des Endoskops übertragen. Im zweiten Fall ist das vom Objekt remittierte Licht am proximalen Ende des Endoskops über ein Okular beobachtbar oder wird mittels einer Kamera erfasst. Alternativ oder zusätzlich zu remittiertem Licht kann auch vom Objekt emittiertes Licht beobachtet werden, insbesondere Fluoreszenzlicht.

Die Qualität eines mittels eines Endoskopiesystems erfassten Bilds, insbesondere Helligkeit, Helligkeits- und Farbkontrast, Signal-Rausch-Abstand, Farbtreue und Auflösung bzw. Schärfe, hängt vom betrachteten Objekt, insbesondere seinen optischen Eigenschaften, und vor allem vom Endoskopiesystem ab. Relevante Faktoren sind beispielsweise die Funktionsfähigkeit der Lichtquelle, ihre Strahlungsleistung bzw. der von ihr erzeugte Lichtstrom, das Spektrum des erzeugten Lichts, ggf. die Übertragungseigenschaften eines verwendeten Lichtleitkabels und der Kopplung des Lichtleitkabels mit der Lichtquelle und dem Endoskop, die Funktionsfähigkeit der Lichtübertragung innerhalb des Endoskops, der Wirkungsgrad der Auskopplung des Lichts der Lichtquelle aus dem Endoskop, die Funktionsfähigkeit bzw. die optischen Eigenschaften des Beobachtungs-Strahlengangs im Endoskop, ggf. einschließlich eines geordneten Bündels von Lichtwellenleitern oder einer Stablinsenoptik, die Funktionsfähigkeit des Okulars oder der Kamera. Häufige Fehlerquellen bilden u. a. die einem Alterungsprozess unterworfene Lichtquelle, ggf. das Lichtleitkabel und seine Kopplung an die Lichtquelle und das Endoskop und die Kopplung einer Kamera an das Endoskop.

Insbesondere für medizinisch-diagnostische Zwecke wird Fluoreszenzlicht beobachtet. In der Photodynamischen Diagnostik (PDD) wird beispielsweise eine durch verabreichtes 5-Aminolävulinsäure (ALA) induzierte Fluoreszenz von Protoporphyrin IX beobachtet. Die Anreicherung von ALA und damit auch die Intensität der Fluoreszenz sind vom Zustand des Gewebes abhängig. Bei der Autofluoreszenz-Diagnostik (AF-Diagnostik) wird die Fluoreszenz von körpereigenen Fluorophoren beobachtet, deren Konzentration ebenfalls vom Zustand des Gewebes abhängig ist. Auch außerhalb der Medizin werden fluoreszenzdiagnostische Verfahren verwendet.

Damit remittiertes Anregungslicht bzw. Beleuchtungslicht die Fluoreszenz nicht überstrahlt, werden im Beleuchtungs- bzw. Anregungs-Strahlengang zwischen Lichtquelle und Objekt ein Beleuchtungsfilter und im Beobachtungs-Strahlengang zwischen Objekt und Kamera bzw. Okular ein Beobachtungsfilter verwendet. Das Beleuchtungsfilter ist ein Kurzpassfilter, das im wesentlichen nur die zur Anregung der Fluoreszenz erforderlichen kurzen Wellenlängen transmittiert, längere Wellenlängen hingegen überwiegend oder fast ausschließlich reflektiert oder absorbiert. Eine sehr geringe, aber nicht verschwindende Transmission im Blockbereich ist bei manchen Anwendungen erwünscht, um auch ohne Fluoreszenz ein Bild zu erhalten, das eine geringe Helligkeit aufweist aber sichtbar ist. Das Beobachtungsfilter ist ein Langpassfilter, das nur Wellenlängen der Fluoreszenz transmittiert und vom Objekt remittiertes kurzwelliges Anregungslicht reflektiert oder absorbiert. Beleuchtungs- bzw. Anregungsfilter können in der Regel manuell oder maschinell ausgetauscht bzw. gewechselt werden. Beobachtungsfilter können austauschbar bzw. wechselbar sein, sind aber in vielen Fällen fest in das Endoskop eingebaut. Beispielsweise in der Urologie werden für die Beobachtung in Weißlicht, ALA- oder AF-Fluoreszenz verschiedene Endoskope verwendet, die zumindest im Beobachtungs-Strahlengang für ihre jeweilige Verwendung optimiert sind bzw. eine entsprechende Filtercharakteristik aufweisen. Zu den oben genannten Fehlerquellen bzw. Einflüssen auf die Funktionsfähigkeit des Endoskopiesystems tritt im Fall der Beobachtung von Fluoreszenz somit noch die Kombination des Beleuchtungsfilters bzw. des Spektrums der Lichtquelle einerseits und des Beobachtungsfilters andererseits.

Eine entsprechende Problemstellung existiert bei anderen optischen Untersuchungssystemen, die eine bildgebende Einrichtung und eine Lichtquelle zur optischen Untersuchung medizinischer und nicht-medizinischer Objekte in remittiertem Licht und/oder in Fluoreszenzlicht umfassen. Dazu zählen Exoskope, die beispielsweise zur Diagnostik und für mikrochirurgische Eingriffe an oder nahe Körperoberflächen verwendet werden.

In der DE 196 38 809 A1 ist eine Vorrichtung zur Prüfung und/oder Justierung eines PDD- oder PDT-Systems (PDT = Photodynamische Therapie) und/oder zur Schulung an einem derartigen System beschrieben. In einem Gehäuse ist ein Target angeordnet, dem gegenüber ein distales Ende eines Endoskops angeordnet werden kann. Die Krümmung des Targets kann der Objektfeldkrümmung einer bildgebenden Einheit des Endoskops entsprechen. Im Target sind ein Fotoelement und Lichtquellen vorgesehen. Das Fotoelement erfasst die Beleuchtungsstärke eines vom Endoskop ausgehenden Beleuchtungslichts. Eine Steuerung steuert die Lichtquellen als Funktion der vom Fotoelement erfassten Beleuchtungsstärke.

In der DE 198 55 853 A1 ist eine Vorrichtung zur Prüfung und/oder Justierung eines PDD- oder PDT-Systems und/oder zur Schulung an einem derartigen System beschrieben. Die Vorrichtung umfasst ein Lumineszenzphantom mit einem Fluoreszenzfarbstoff. Gegenüber dem Lumineszenzphantom kann ein Ende eines Endoskops angeordnet werden.

In der nachveröffentlichten DE 10 2009 043 696 sind eine Vorrichtung und ein Verfahren zum Prüfen von Endoskopen beschrieben. Die Vorrichtung umfasst ein Filtermodul mit mehreren Durchbrüchen, in denen optische Filter angeordnet sind. Das Filtermodul wird aus einer Richtung durch die Lichtquelle über ein Lichtleitkabel beleuchtet. Aus einer entgegengesetzten Richtung wird das von dem Filtermodul transmittierte Licht mittels eines Endoskops beobachtet.

In US 2003/0105195 A1 sind eine Vorrichtung zum Kalibrieren eines optischen Scanners und ein Verfahren zu dessen Verwendung beschrieben. Im Mittelpunkt stehen die Kalibrierung optischer Scanner für fluoreszente Proben und die Kalibrierung der Empfindlichkeit eines optischen Detektors, des Abstands zwischen einem Objekttisch und einem Objektiv, der Geschwindigkeit der Bewegung des Objekttisches, des Scannerspiegels. Die Vorrichtung zum Kalibrieren umfasst ein Substrat mit einer Polymerschicht mit einem fluoreszenten Agens. Die Oberfläche der Vorrichtung zum Kalibrieren wird mit Strahlung zur Anregung von Fluoreszenz des fluoreszenten Agens bestrahlt, wobei Fluoreszenzdaten erhalten werden, mittels derer der Scanner kalibriert wird.

Abhängig von konkreten, in der Praxis sich ergebenden Aufgabenstellungen kann jede der bislang bekannten Vorrichtungen und Verfahren Vor- und Nachteile aufweisen. Beispielsweise ermöglicht unter einigen Bedingungen und für einige Anwendungen keine der beschriebenen Vorrichtungen und Verfahren eine zuverlässige und evtl. sogar quantitative Prüfung eines vollständigen Endoskopiesystems oder eines vollständigen anderen optisehen Untersuchungssystems in genau dem Zustand, in dem es davor oder danach medizinisch oder nicht-medizinisch verwendet wurde oder wird.

Eine Aufgabe der vorliegenden Erfindung besteht darin, eine verbesserte Prüfvorrichtung und ein verbessertes Verfahren zum Prüfen eines optischen Untersuchungssystems zu schaffen, die insbesondere eine absolute und genaue Prüfung der Funktionsfähigkeit oder einer anderen Eigenschaft des optischen Untersuchungssystems vereinfachen.

Diese Aufgabe wird durch die Gegenstände der unabhängigen Ansprüche gelöst.

Weiterbildungen sind in den abhängigen Ansprüchen angegeben.

Ausführungsformen der vorliegenden Erfindung beruhen auf der Idee, bei einer Prüfvorrichtung für ein optisches Untersuchungssystem mit einer bildgebenden Einrichtung und einer Lichtquelle eine Positionierungseinrichtung zum Halten der bildgebenden Einrichtung bei einer vorbestimmten Position und insbesondere auch einer vorbestimmten Richtung des distalen Endes der bildgebenden Einrichtung zu einer Referenzoberfläche der Prüfvorrichtung vorzusehen. Dazu weist die Positionierungsvorrichtung insbesondere einen mechanischen Anschlag auf, der so ausgebildet ist, dass das distale Ende einer in die Prüfvorrichtung eingesetzten bildgebenden Einrichtung an einer vorbestimmten Position, insbesondere auch einem vorbestimmten Abstand zur Referenzoberfläche, liegt, wenn die bildgebende Einrichtung an dem Anschlag anliegt.

Die Positionierungseinrichtung ermöglicht auf einfache Weise eine reproduzierbare und genaue Positionierung des distalen Endes der bildgebenden Einrichtung relativ zur Referenzoberfläche der Prüfvorrichtung. Die Prüfvorrichtung kann damit nicht nur für Schulungszwecke oder eine qualitative Messung oder Prüfung der Funktionsfähigkeit eines optischen Untersuchungssystems verwendet werden, sondern auch für eine quantitative Prüfung. Durch die präzise und reproduzierbare Positionierung des distalen Endes der bildgebenden Einrichtung relativ zu der Referenzoberfläche der Prüfvorrichtung können beispielsweise aus der Helligkeit eines von der bildgebenden Einrichtung erzeugten Bilds der Referenzoberfläche quantitative Rückschlüsse auf die Funktionsfähigkeit und andere Eigenschaften des optischen Untersuchungssystems gezogen werden. Wenn das von der bildgebenden Einrichtung erzeugte Bild der Referenzoberfläche zu dunkel ist, kann das nicht mehr an einer falschen Positionierung des distalen Endes der bildgebenden Einrichtung relativ zur Referenzoberfläche liegen. Vielmehr muss ein Defekt an dem optischen Untersuchungssystem vorliegen, beispielsweise eine nicht mehr oder nur noch eingeschränkt funktionsfähige Lichtquelle, ein defektes Lichtleitkabel oder eine fehlerhafte Kopplung zwischen einem Lichtleitkabel und der Lichtquelle oder der bildgebenden Einrichtung.

Eine Prüfvorrichtung für ein optisches Untersuchungssystem mit einer bildgebenden Einrichtung und einer Lichtquelle zur optischen Untersuchung eines Objekts in remittiertem Licht und/oder in Fluoreszenzlicht umfasst ein Gehäuse mit einem Hohlraum und einer Öffnung zum Einführen eines distalen Endes der bildgebenden Einrichtung in den Hohlraum, eine Referenzoberfläche mit vorbestimmten optischen Eigenschaften in dem Hohlraum zumindest entweder zur Remission von auf die Referenzoberfläche fallendem Beleuchtungslicht oder zur Emission von Fluoreszenzlicht, und eine Positionierungseinrichtung zum Halten der bildgebenden Einrichtung bei einer vorbestimmten Position des distalen Endes der bildgebenden Einrichtung relativ zur Referenzoberfläche.

Die Prüfvorrichtung ist insbesondere eine Prüfvorrichtung für ein Endoskopiesystem mit einem Endoskop und einer Lichtquelle. Die Lichtquelle kann in das Endoskop integriert sein oder als separate Einheit vorliegen. Im zweiten Fall kann die Lichtquelle, beispielsweise mittels eines Lichtleitkabels, mit der bildgebenden Einrichtung gekoppelt sein, wobei die bildgebende Einrichtung zur Übertragung von Beleuchtungslicht der Lichtquelle zum distalen Ende der bildgebenden Einrichtung ausgebildet ist. Alternativ kann das optische Untersuchungssystem ausgebildet sein, um Beleuchtungslicht der Lichtquelle über einen Beleuchtungs-Strahlengang außerhalb der bildgebenden Einrichtung zu einem zu untersuchenden bzw. zu betrachtenden Objekt zu leiten.

Die Referenzoberfläche kann eben oder gekrümmt, insbesondere konkav sein. Die vorbestimmten optischen Eigenschaften der Referenzoberfläche sind insbesondere zeitlich unveränderlich bzw. stabil. Zu den optischen Eigenschaften der Referenzoberfläche zählen insbesondere ihr Remissionsgrad als Funktion der Wellenlänge von Beleuchtungslicht und ggf. eine Fluoreszenz-Quantenausbeute als Funktion der anregenden Wellenlänge und der Wellenlänge des emittierten Fluoreszenzlichts. Hinsichtlich ihrer Remissionseigenschaften ist die Referenzoberfläche beispielsweise näherungsweise ein Lambert-Strahler mit einer näherungsweise ideal diffusen Remission.

Die Positionierungseinrichtung kann austauschbar sein, um die Prüfvorrichtungen mit unterschiedlichen bildgebenden Einrichtungen betreiben zu können. Ferner kann die Positionierungseinrichtung ausgebildet sein, um zumindest dann, wenn eine bildgebende Einrichtung in die Prüfvorrichtung eingesetzt ist, den Hohlraum des Gehäuses der Prüfvorrichtung gegen den Einfall von Umgebungslicht abzuschirmen. Die durch die Positionierungseinrichtung vorbestimmte Position des distalen Endes der bildgebenden Einrichtung relativ zu der Referenzoberfläche der Prüfvorrichtung, insbesondere der Abstand zwischen beiden, kann so gewählt sein, dass die relative Position einer typischen Position des distalen Endes der bildgebenden Einrichtung bei einer optischen Untersuchung eines Objekts entspricht. Die Prüfvorrichtung ermöglicht somit eine Prüfung eines optischen Untersuchungssystems unter realistischen Bedingungen.

Um eine Prüfung eines optischen Untersuchungssystems unter möglichst realistischen Bedingungen zu ermöglichen, kann die Prüfvorrichtung ferner ausgebildet sein, um Wasser oder ein anderes Fluid zu enthalten, dessen Brechungsindex größer als 1 ist. Dazu sind die Prüfvorrichtung und insbesondere das Gehäuse und die Positionierungseinrichtung so ausgebildet, dass das Fluid zumindest dann nicht oder nur langsam entweicht, wenn eine bildgebende Einrichtung in die Prüfvorrichtung eingesetzt ist. Das Gehäuse und die Positionierungseinrichtung umfassen dazu beispielsweise O-Ringe oder andere Dichtungen. Wenn die Prüfvorrichtung dazu ausgebildet ist, eine Flüssigkeit zu enthalten, kann die Öffnung zum Hohlraum im Gehäuse in einem oberen Bereich angeordnet sein, insbesondere am höchsten Punkt des Hohlraums. Dies ermöglichst beispielsweise eine Simulation der Bedingungen, die in der Urologie bei einer endoskopischen Untersuchung einer mit Harn gefüllten Harnblase vorliegen.

Die Positionierungseinrichtung kann ferner dazu ausgebildet sein, eine Rotation einer in die Prüfvorrichtung eingesetzten bildgebenden Einrichtung um ihre Längsachse zu unterbinden. Dazu umfasst die Positionierungseinrichtung insbesondere eine Einrichtung zum kraftschlüssigen oder formschlüssigen Unterbinden einer Rotation einer bildgebenden Einrichtung, beispielsweise eine Kugel-Rastvorrichtung. Diese Einrichtung kann gleichzeitig dazu ausgebildet sein, eine in die Prüfvorrichtung eingesetzte bildgebende Einrichtung, insbesondere ihr distales Ende, an der vorbestimmten Position zu halten. Das Unterbinden einer Rotation einer bildgebenden Einrichtung in der Prüfvorrichtung um ihre Längsachse kann insbesondere bei nicht-axialer Blickrichtung der bildgebenden Einrichtung vorteilhaft sein, um sicherzustellen, dass die bildgebende Einrichtung auf die Referenzoberfläche oder gegebenenfalls auf ein bestimmtes Merkmal, beispielsweise eine Marke, an der Referenzoberfläche gerichtet ist.

Eine Prüfvorrichtung, wie sie hier beschrieben ist, kann so ausgebildet sein, dass die Positionierungseinrichtung ohne Verwendung von Werkzeug von dem Gehäuse der Prüfvorrichtung getrennt und mit diesem wieder verbunden werden kann. Dazu ist insbesondere am Gehäuse der Prüfvorrichtung eine Kugel-Rasteinrichtung vorgesehen, die in eine Ausnehmung oder Hinterschneidung an der Positionierungseinrichtung eingreifen kann, um die Positionierungseinrichtung an dem Gehäuse der Prüfvorrichtung zu halten. Dies ermöglicht nicht nur eine unaufwendige und schnelle Zerlegung der Prüfvorrichtung zur Reinigung und Sterilisierung, sondern auch einen schnellen Austausch der Positionierungseinrichtung zur Anpassung der Prüfvorrichtung an unterschiedliche bildgebende Einrichtungen.

Eine Prüfvorrichtung, wie sie hier beschrieben ist, insbesondere eine Prüfvorrichtung bei der die Positionierungseinrichtung ohne Verwendung von Werkzeug von dem Gehäuse getrennt werden kann, kann am Gehäuse und an der Positionierungseinrichtung Einrichtungen zum formschlüssigen Unterbinden einer Rotation der Positionierungseinrichtung um ihre Längsachse relativ zu dem Gehäuse umfassen. Solche Einrichtungen umfassen beispielsweise einen Stift oder eine andere konvexe Einrichtung am Gehäuse, der oder die in eine Längsnut oder eine andere konkave Einrichtung an der Positionierungseinrichtung eingreift. Dies unterstützt eine definierte Ausrichtung des distalen Endes der bildgebenden Einrichtung relativ zur Referenzoberfläche. Die definierte Ausrichtung des distalen Endes der bildgebenden Einrichtung relativ zur Referenzoberfläche kann unter anderem vorteilhaft sein im Fall einer nicht-kugelförmigen Referenzoberfläche und im Fall von Marken oder anderen Merkmalen an der Referenzoberfläche, auf welche die bildgebende Einrichtung zu richten ist.

Eine Prüfvorrichtung, wie sie hier beschrieben ist, insbesondere eine Prüfvorrichtung, bei der die Positionierungseinrichtung ohne Verwendung von Werkzeug von dem Gehäuse getrennt und wieder mit ihm verbunden werden kann, kann eine Rasteinrichtung, insbesondere eine Kugel-Rasteinrichtung, umfassen, die dazu ausgebildet ist, die Positionierungseinrichtung lösbar an der Öffnung bzw. am Gehäuse der Prüfvorrichtung zu befestigen.

Die Positionierungseinrichtung einer Prüfvorrichtung, wie sie hier beschrieben ist, kann an ihrem vom Hohlraum abgewandten Ende eine Halteeinrichtung, insbesondere eine Kugelraste, zum rastenden Halten des proximalen Endes einer in die Prüfvorrichtung eingesetzten bildgebenden Einrichtung umfassen. Diese Halteeinrichtung ist beispielsweise eine Kugel-Rasteinrichtung, die in eine Ausnehmung oder Hinterschneidung am proximalen Ende einer bildgebenden Einrichtung eingreifen kann. Einige bildgebende Einrichtungen, insbesondere Endoskope, weisen von Haus aus eine derartige Ausnehmung bzw. Hinterschneidung für andere Zwecke auf. Insbesondere bei einem Endoskop mit einem Schaft mit einer definierten oder standardisierten Länge kann eine Festlegung der Position des proximalen Endes der bildgebenden Einrichtung zwangsläufig eine vorbestimmte Position des distalen Endes der bildgebenden Einrichtung zur Folge haben. Dabei muss die bildgebende Einrichtung an ihrem distalen Ende keine Merkmale aufweisen, die ein formschlüssiges Halten der bildgebenden Einrichtung ermöglichen, sondern kann beispielsweise eine einfache kreiszylindrische Form aufweisen.

Die Referenzoberfläche ist insbesondere so ausgebildet, dass für eine oder mehrere verschiedene Blickrichtungen einer in die Prüfvorrichtung eingesetzten bildgebenden Einrichtung die Tangentialfläche an die Referenzoberfläche im Schnittpunkt der Blickrichtung mit der Referenzoberfläche jeweils senkrecht zur Blickrichtung steht. Ferner kann die Referenzoberfläche so ausgebildet sein, dass für mehrere verschiedene Blickrichtungen der Abstand zwischen der vorbestimmten Position des distalen Endes der bildgebenden Einrichtung und dem jeweiligen Schnittpunkt der Blickrichtung mit der Referenzoberfläche gleich ist. Die Blickrichtung ist die Richtung vom distalen Ende der bildgebenden Einrichtung aus, in der ein Gegenstand liegt, der in der Mitte eines durch die bildgebenden Einrichtung erzeugten Bilds liegt.

Die Referenzoberfläche kann einen Ausschnitt aus einer Kugeloberfläche bilden, wobei die für das distale Ende der bildgebenden Einrichtung vorgesehene Position am Mittelpunkt der Kugeloberfläche liegt. Mit dem distalen Ende der bildgebenden Einrichtung ist in diesem Zusammenhang insbesondere der objektseitige Hauptpunkt bzw. der Schnittpunkt der optischen Achse mit der objektseitigen Hauptebene der bildgebenden Einrichtung gemeint. Bei einer kugelflächenförmigen Ausbildung der Referenzoberfläche kann hingenommen werden, dass eine in die Prüfvorrichtung eingesetzte bildgebende Einrichtung um ihre Längsachse frei rotierbar ist. Insbesondere muss in diesem Fall die Positionierungseinrichtung nicht dazu ausgebildet sein, eine Rotation der bildgebenden Einrichtung zu unterbinden.

Alternativ kann die Referenzoberfläche einen Ausschnitt aus einer Kreiszylinderfläche bilden, wobei die für das distale Ende der bildgebenden Einrichtung vorgesehene Position an der Symmetrieachse der Kreiszylinderfläche liegt. Die Krümmung bzw. der Radius der Referenzoberfläche kann an die Objektfeldkrümmung der bildgebenden Einrichtung angepasst sein, die wiederum typischerweise an die vorgesehene Anwendung des optischen Untersuchungssystems angepasst ist. Der Radius der Kugeloberfläche bzw. der Kreiszylinderfläche beträgt deshalb für viele Anwendungen höchstens 100 mm und liegt insbesondere im Bereich zwischen 10 mm und 50 mm oder auch im Bereich von 5 mm oder darunter, bis hin zur Kontaktendoskopie. Kugeloberflächen und Kreiszylinderflächen können mit vergleichsweise geringem Aufwand erzeugt werden und ermöglichen einen definierten Abstand der Referenzoberfläche vom distalen Ende der bildgebenden Einrichtung auch bei Verwendung der Prüfvorrichtung für bildgebende Einrichtungen mit unterschiedlichen Blickrichtungen.

Wenn die Referenzoberfläche einen Ausschnitt aus einer Kugeloberfläche bildet, kann die Referenzoberfläche so ausgebildet sein, dass zwei gegenüberliegende Orte am Rand der Referenzoberfläche auf einer Geraden liegen, die einen Winkel zwischen 40 und 80 Grad zur Längsachse einer von der Positionierungseinrichtung gehaltenen bildgebenden Einrichtung bildet. Insbesondere liegt der Winkel im Bereich von 50 Grad bis 70 Grad. Wenn die Referenzoberfläche einen Ausschnitt aus einer Kugeloberfläche bildet, insbesondere halbkugelförmig ist, kann der Rand der Referenzoberfläche in einer Ebene liegen, deren Normale einen Winkel im Bereich von 10 Grad bis 50 Grad zur Längsachse einer von der Positionierungseinrichtung gehaltenen bildgebenden Einrichtung bildet. Der Winkel zwischen der Normalen der Ebene und der Längsachse der Positionierungseinrichtung liegt insbesondere im Bereich von 20 Grad bis 40 Grad, beispielsweise bei 30 Grad. Wenn die Referenzoberfläche einen Ausschnitt aus einer Kreiszylinderfläche bildet, bildet eine Gerade durch zwei gegenüberliegende Orte am Rand der Referenzoberfläche oder eine Ebene, die zwei gegenüberliegende gerade Abschnitte des Rands der Referenzoberfläche enthält, einen Winkel zwischen 40 Grad und 80 Grad mit der Längsachse einer von der Positionierungseinrichtung gehaltenen bildgebenden Einrichtung. Der Winkel liegt insbesondere im Bereich von 50 Grad bis 70 Grad, beispielsweise bei 60 Grad.

Eine Prüfvorrichtung mit einer so ausgestalteten Referenzoberfläche ist zum Prüfen von optischen Untersuchungssystemen mit bildgebenden Einrichtungen mit unterschiedlichen Blickrichtungen geeignet. Die Prüfvorrichtung kann somit beispielsweise für die meisten Endoskope mit einer großen Vielzahl von verschiedenen Blickrichtungen verwendet werden, ohne dass die Referenzoberfläche ausgetauscht werden müsste.

Eine Prüfvorrichtung, wie sie hier beschrieben ist, ist insbesondere zumindest teilweise zerlegbar und vollständig sterilisierbar. Insbesondere sind auch die Referenzoberfläche und die Positionierungseinrichtung uneingeschränkt sterilisierbar. Eine bildgebende Einrichtung kann deshalb noch mittels der Prüfvorrichtung geprüft werden, unmittelbar bevor die bildgebende Einrichtung medizinisch verwendet wird und ohne sie nach der Prüfung nochmals sterilisieren zu müssen.

Die Referenzoberfläche einer Prüfvorrichtung, wie sie hier beschrieben ist, ist weiß oder weist einen für einen Weißabgleich ausreichend großen weißen Bereich auf. Dies bedeutet insbesondere, dass der Remissionsgrad der Referenzoberfläche oder ihres weißen Bereichs im für das menschliche Auge sichtbaren Wellenlängebereich - höchstens von kleinen Wellenlängenbereichen abgesehen - hoch ist, beispielsweise mindestens 80 % beträgt. Insbesondere ist die Referenzoberfläche eine Oberfläche eines Körpers aus gefülltem Polytetrafluorethylen oder Silikon. Die Referenzoberfläche ist damit auch zur Durchführung eines Weißabgleichs geeignet. Polytetrafluorethylen wird u. a. unter der Bezeichnung Teflon von DuPont vertrieben, weist stabile bzw. zeitlich unveränderliche optische Eigenschaften auf und kann ohne Weiteres autoklaviert werden.

Alternativ ist die Referenzoberfläche grau oder farbig und/oder weist einen oder mehrere graue oder farbige Bereiche auf. Der Remissionsgrad und/oder die Farbe und/oder Fluoreszenzeigenschaften der Referenzoberfläche oder von Teilen derselben können an typische oder durchschnittliche Remissionsgrade oder Farbtöne bzw. Farbwerte oder Fluoreszenzeigenschaften relevanter zu untersuchender Objekte angepasst sein. Insbesondere können die optischen Eigenschaften der Referenzoberfläche oder eines oder mehrerer Bereiche derselben den mittleren optischen Eigenschaften von biologischem Gewebe nachgebildet sein. Ferner können die optischen Eigenschaften der Referenzoberfläche strukturiert sein, um beispielsweise Blutgefäße oder andere Strukturen von biologischem Gewebe nachzubilden.

Bei einem Verfahren zum Prüfen eines optischen Untersuchungssystems mit einer bildgebenden Einrichtung und einer Lichtquelle zur optischen Untersuchung eines Objekts in remittiertem Licht und/oder Fluoreszenzlicht wird das distale Ende der bildgebenden Einrichtung durch eine Öffnung in einen Hohlraum in einem Gehäuse einer Prüfvorrichtung eingeführt. Das distale Ende der bildgebenden Einrichtung wird beim Einführen oder danach an einer vorbestimmten Position relativ zu einer Referenzoberfläche mit vorbestimmten optischen Eigenschaften in dem Hohlraum angeordnet. Die Referenzoberfläche wird mittels der bildgebenden Einrichtung mit Beleuchtungslicht mit einem vorbestimmten Beleuchtungsspektrum beleuchtet. Das durch die Referenzoberfläche remittierte Licht wird mittels der bildgebenden Einrichtung erfasst. Die Funktionsfähigkeit oder eine andere Eigenschaft des optischen Untersuchungssystems wird anhand des erfassten remittierten Lichts bestimmt.

Das Prüfverfahren wird insbesondere mit einer der hier beschriebenen Prüfvorrichtungen durchgeführt.

### Kurzbeschreibung der Figuren

Nachfolgend werden Ausführungsformen anhand der beigefügten Figuren näher erläutert. Es zeigen:
- Figur 1: eine schematische Darstellung eines optischen Untersuchungssystems;
- Figur 2: eine schematische Darstellung eines Endoskops mit einer Prüfvorrichtung;
- Figur 3: eine schematische Darstellung einer Prüfvorrichtung;
- Figur 4: eine schematische Darstellung einer Prüfvorrichtung mit einem Endoskop;
- Figur 5: eine schematische Darstellung einer Prüfvorrichtung;
- Figur 6: eine weitere schematische Darstellung der Prüfvorrichtung aus Figur 5;
- Figur 7: eine schematische Darstellung mehrerer Spektren;
- Figur 8: eine schematische Darstellung von Produkten von Transmissionsspektren;
- Figur 9: eine schematische Darstellung von Weißabgleich-Parametern;
- Figur 10: eine schematische Darstellung weiterer Weißabgleich-Parameter;
- Figur 11: ein schematisches Flussdiagramm.

### Beschreibung der Ausführungsformen

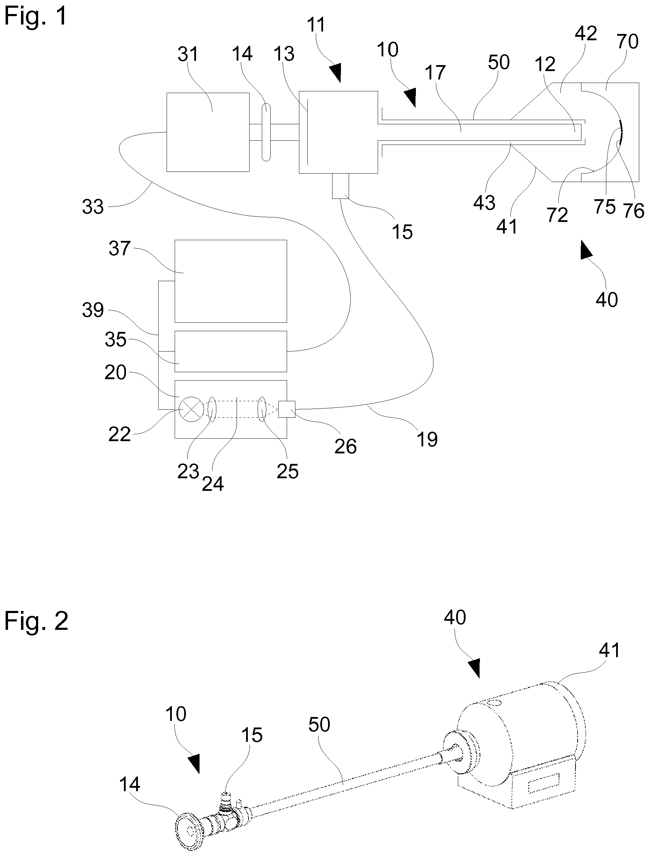

Figur 1 zeigt eine schematische Darstellung eines optischen Untersuchungssystems. Das optische Untersuchungssystem ist in diesem Beispiel ein Endoskopiesystem, das beispielsweise bei medizinisch-diagnostischen Verfahren in der Urologie und in anderen Fachgebieten einsetzbar ist. Das Endoskopiesystem umfasst ein Endoskop 10 mit einem proximalen Ende 11 und einem distalen Ende 12. Das Endoskop 10 umfasst einen Beleuchtungs- bzw. Anregungs-Strahlengang und einen Beobachtungs-Strahlengang, die in Figur 1 nicht im Detail dargestellt sind. Der Beleuchtungs-Strahlengang umfasst insbesondere einen oder mehrere Lichtwellenleiter zum Übertragen von Beleuchtungs- bzw. Anregungslicht vom proximalen Ende 11 zum distalen Ende 12 und einen Lichtaustritt am distalen Ende 12, durch den Beleuchtungslicht aus dem distalen Ende 12 des Endoskops 10 austreten kann, um ein zu betrachtendes Objekt zu beleuchten. Der Beobachtungs-Strahlengang umfasst einen Lichteintritt am distalen Ende 12 des Endoskops 10, eine Optik zum Übertragen von Beobachtungslicht, das von einem betrachteten Objekt ausgeht, vom distalen Ende 12 zum proximalen Ende 11, ein Beobachtungsfilter 13 und ein Okular 14. Zum Übertragen des Beobachtungslichts vom distalen Ende 12 zum proximalen Ende 11 des Endoskops 10 ist beispielsweise eine Stablinsenoptik oder ein geordnetes Bündel von Lichtwellenleitern in einem Schaft 17 des Endoskops 10 vorgesehen. Das Endoskop 10 weist ferner am proximalen Ende 11 eine Kupplung 15 zum mechanischen und optischen Koppeln eines Lichtleitkabels 19 mit dem beschriebenen Beleuchtungs-Strahlengang im Endoskop 10 auf.

Das Endoskop 10 ist über das Lichtleitkabel 19 mit einer Lichtquellenvorrichtung 20 gekoppelt. Die Lichtquellenvorrichtung 20 umfasst eine Lichtquelle 22, beispielsweise eine Halogen-Glühlampe, eine Hochdruck-Gasentladungslampe, eine Leuchtdiode oder einen Laser. Ferner umfasst die Lichtquellenvorrichtung 20 eine erste Sammellinse 23, ein Beleuchtungsfilter 24 und eine zweite Sammellinse 25. Die Lichtquelle 22 ist über die erste Sammellinse 23, das Beleuchtungsfilter 24, die zweite Sammellinse 25 und eine Kupplung 26 mit dem Lichtleitkabel 19 gekoppelt.

Eine Kamera 31 ist über das Okular 14 mit dem Endoskop 10 und dessen Beobachtungs-Strahlengang mechanisch bzw. optisch gekoppelt. Die Kamera 31 umfasst einen lichtempfindlichen Bildsensor, beispielsweise einen CCD- oder CMOS-Sensor, zum Wandeln von auf den Bildsensor fallendem Licht in analoge oder digitale elektrische Signale. Die Kamera 31 ist über ein Signalkabel 33 zur Übertragung analoger oder digitaler elektrischer oder optischer Signale mit einer Kamerasteuerung 35 gekoppelt, die auch als CCU = camera control unit bezeichnet wird.

Die Lichtquellenvorrichtung 20, die Kamerasteuerung 35 und ein Bildschirm 37 sind über einen Kommunikationsbus 39 oder mehrere separate Signalleitungen miteinander gekoppelt. Über den Kommunikationsbus 39 können weitere, in Figur 1 nicht dargestellte Vorrichtungen innerhalb oder außerhalb des Behandlungsraums, in dem das Endoskopiesystem angeordnet ist, mit der Lichtquellenvorrichtung 20, der Kamerasteuerung 35 und dem Bildschirm 37 gekoppelt sein, beispielsweise eine Datenbank, eine Tastatur, eine Computermaus und andere Benutzerschnittstellen.

In Figur 1 ist ferner eine Prüfvorrichtung 40 mit einem lichtdichten Gehäuse 41, einem Hohlraum 42 in dem lichtdichten Gehäuse 41 und einer Öffnung 43 zum Hohlraum 42 dargestellt. Das distale Ende 12 des Endoskops 10 ist durch die Öffnung 43 in den Hohlraum 42 der Prüfvorrichtung 40 eingeführt. Eine in der Öffnung 43 angeordnete Positionierungseinrichtung 50 hält form- und/oder kraftschlüssig den Schaft 17 des Endoskops 10 so, dass das distale Ende 12 des Endoskops 10 an einer vorbestimmten Position und in einer vorbestimmten Richtung im Hohlraum 42 angeordnet ist. Ferner verhindert die Positionierungseinrichtung 50, zumindest wenn der Schaft 17 des Endoskops 10 in der Positionierungseinrichtung 50 angeordnet ist, weitgehend das Eindringen von Licht aus der Umgebung durch die Öffnung 43 in den Hohlraum 42 im Gehäuse 41.

Im Hohlraum 42 der Prüfvorrichtung 40 ist ferner ein Referenzkörper 70 mit einer Referenzoberfläche 72 angeordnet. Die Referenzoberfläche 72 weist vorbestimmte optische Eigenschaften und die räumliche Gestalt eines Ausschnitts aus einer Kugeloberfläche oder aus einem Kreiszylindermantel auf. Die für das distale Ende 12 des Endoskops 10 vorgesehene Position liegt insbesondere am Mittelpunkt dieser Kugeloberfläche bzw. an der Symmetrieachse des Kreiszylindermantels. Insbesondere liegt der objektseitige Hauptpunkt bzw. der Schnittpunkt der optischen Achse mit der objektseitigen Hauptebene der bildgebenden Einrichtung 10 am Mittelpunkt der Kugeloberfläche bzw. an der Symmetrieachse des Kreiszylindermantels.

Die Referenzoberfläche 72 weist zeitlich unveränderliche bzw. stabile vorbestimmte optische Eigenschaften auf. Die Referenzoberfläche 72 kann weiß sein bzw. einen Remissionsgrad aufweisen, der im für das menschliche Auge sichtbaren Spektralbereich im Wesentlichen wellenlängenunabhängig ist. Die Referenzoberfläche 72 kann alternativ farbig sein bzw. einen im für das menschliche Auge sichtbaren Spektralbereich wellenlängenabhängigen Remissionsgrad aufweisen. Alternativ oder zusätzlich kann die Referenzoberfläche 72 fluoreszierend sein. Dabei liegen die zur Anregung der Fluoreszenz erforderlichen Wellenlängen beispielsweise im ultravioletten oder, für medizinische Anwendungen bevorzugt, im blauen Spektralbereich und das emittierte Fluoreszenzlicht im grünen, roten oder infraroten Spektralbereich. Die optischen Eigenschaften können über die gesamte Referenzoberfläche 72 homogen bzw. ortsunabhängig sein.

Alternativ weist die Referenzoberfläche mehrere Bereiche mit verschiedenen optischen Eigenschaften auf. Bei dem in Figur 1 dargestellten Beispiel ist die Referenzoberfläche 72 überwiegend weiß mit einem Indikatorbereich 75 und einem Referenzbereich 76, die jeweils vom Rest der Referenzoberfläche 72 verschiedene optische Eigenschaften aufweisen. Der Indikatorbereich 75 und der Referenzbereich 76 können mit scharfen Rändern oder aufgrund ihrer Anordnung oder Gestalt eine Fokussierung oder eine Einstellung der Brennweite bzw. der Größe des Sichtfelds der bildgebenden Einrichtung vereinfachen oder ermöglichen. Darüber hinaus können die optischen Eigenschaften des Indikatorbereichs 75 und des Referenzbereichs 76 eine Bestimmung des Transmissionsspektrums des Beleuchtungsfilters 24 und des Transmissionsspektrums des Beobachtungsfilters 13 vereinfachen.

Der Referenzkörper 70 besteht, von dem Indikatorbereich und dem Referenzbereich 75 an der Referenzoberfläche 72 abgesehen, insbesondere aus Polytetrafluorethylen PTFE, das beispielsweise unter dem Markennamen Teflon von DuPont vertrieben wird, oder aus Silikon. Sowohl PTFE als auch Silikon kann mit weißen oder farbigen Pigmenten oder Farbstoffen gefüllt sein.

Figur 2 zeigt eine schematische axonometrische Darstellung eines Endoskops 10 und einer Prüfvorrichtung 40, die dem Endoskop und der Prüfvorrichtung ähnlich sind, die oben anhand der Figur 1 dargestellt wurden. Im Unterschied zur Figur 1 sind keine separate Lichtquelle, Kamera oder andere Vorrichtungen gezeigt. Die Positionierungseinrichtung 50 erstreckt sich bei dem in Figur 2 gezeigten Beispiel bis zum proximalen Ende 11 des Endoskops 10 und nimmt den gesamten Schaft des Endoskops 10 auf. Die exakte Positionierung des distalen Endes des Endoskops 10 in der Prüfvorrichtung 40 wird bei diesem Beispiel durch Formschluss zwischen der Positionierungseinrichtung 50 und dem distalen Ende 11 des Endoskops 10 erreicht, insbesondere mittels eines mechanischen Anschlags und/oder einer Rastverbindung.

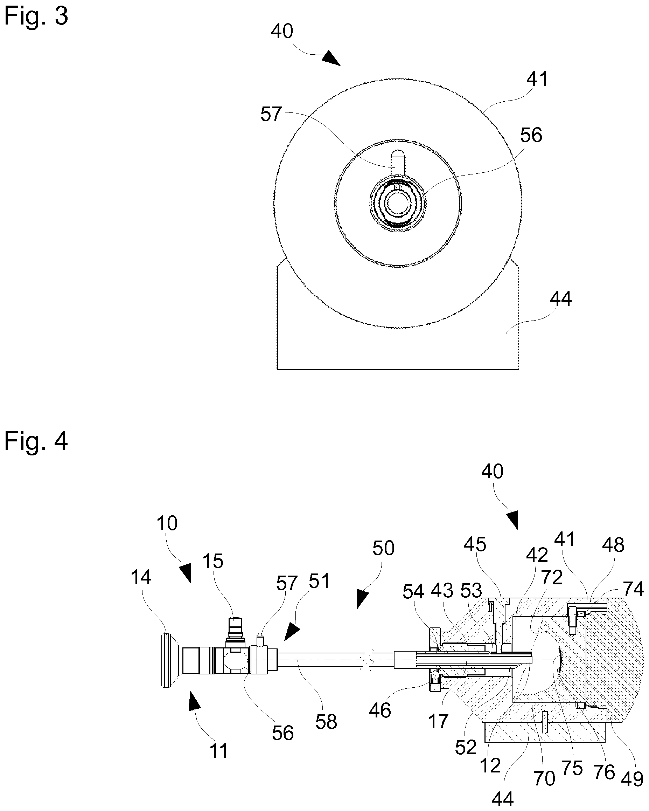

Figur 3 zeigt eine schematische Ansicht der in Figur 2 gezeigten Prüfvorrichtung 40 aus der Richtung, aus der ein Endoskop 10 in die Prüfvorrichtung 40 eingeführt werden kann. Diese Richtung ist parallel zur Längsachse des Schafts 17 des Endoskops 10. Erkennbar ist die kreisförmige Kontur bzw. abschnittsweise kreiszylindrische äußere Form des Gehäuses 41. Um einen sicheren Stand der Prüfvorrichtung 40 zu ermöglichen, ist in das Gehäuse 41 integriert oder mit diesem dauerhaft mechanisch verbunden ein Standfuß 44. Anordnung und Funktion eines Anschlags 56 für ein Endoskop 10 und einer Kugel-Rasteinrichtung 57 sind in Figur 4 besser erkennbar.

Figur 4 zeigt eine weitere schematische Darstellung der in Figur 2 axonometrisch dargestellten Konfiguration aus der Prüfvorrichtung 40 und dem Endoskop 10. Figur 4 zeigt eine Ansicht aus einer Richtung senkrecht zur Längsachse des Schafts des Endoskops 10. Die Prüfvorrichtung 40 ist in einem Längsschnitt dargestellt, wobei die Schnittebene die Längsachse des Schafts des Endoskops 10 enthält.

Deutlich erkennbar in Figur 4 sind das Gehäuse 41, der Hohlraum 42 im Gehäuse 41, die Öffnung 43 zum Hohlraum 42, der Standfuß 44, ein Stift 45, eine Kugel-Rasteinrichtung 46 am äußeren Ende der Öffnung 43, eine Nut 48 und ein Schraubverschluss 49 an der von der Öffnung 43 abgewandten Seite des Gehäuses 41. Ferner ist der Referenzkörper 70 mit der Referenzoberfläche 72 und einem Stift 74 erkennbar. Der Schraubverschluss 49 schließt den Hohlraum 42 ab. Wenn der Schraubverschluss 49 geöffnet ist, kann der Referenzkörper 70 aus dem Hohlraum 42 entnommen werden. Der Hohlraum 42 und der Referenzkörper 70 sind dann mit all ihren Oberflächen in idealer Weise für eine Reinigung und Sterilisierung zugänglich. Beim Einsetzen des Referenzkörpers 70 in den Hohlraum 42 greift der Stift 74 in die Nut 48 ein, so dass der Referenzkörper 70 eine vorbestimmte Lage einnimmt und nicht im Hohlraum 42 rotieren kann.

In Figur 4 ist ferner erkennbar, dass ein Ende 52 der Positionierungseinrichtung 50 durch die Öffnung 43 bis in den Hohlraum 42 der Prüfvorrichtung 40 reicht. Die Positionierungseinrichtung 50 ist im Wesentlichen genauso lang wie der Schaft 17 des Endoskops 10. Die Positionierungseinrichtung 50 führt und hält somit den Schaft 17 des Endoskops über seine gesamte Länge. In der vorgesehenen Position des Endoskops 10 liegt ein Abschnitt der äußeren Oberfläche des Endoskops 10 nahe des proximalen Endes 11 an einem Anschlag 56 an der Positionierungseinrichtung 50 an. Gleichzeitig greift eine in Figur 4 nicht sichtbare federbelastete Kugel der Kugel-Rasteinrichtung 57 in eine Ausnehmung am Endoskop 10 nahe dessen proximalen Ende 11 ein. Damit ist die Position des Endoskops 10 relativ zur Positionierungseinrichtung 50 festgelegt. Abhängig von der Form der Ausnehmung im Endoskop 10, in die die Kugel-Rasteinrichtung 57 am vom Hohlraum 42 der Prüfvorrichtung 40 abgewandten Ende 51 der Positionierungseinrichtung 50 eingreift, kann diese rastende Verbindung gleichzeitig eine Rotation des Endoskops um die Längsachse des Schafts 17 unterbinden.

Das dem Hohlraum 42 der Prüfvorrichtung 40 zugewandte Ende 52 der Positionierungseinrichtung 50 ist im Wesentlichen kreiszylinderförmig und weist gegenüber der entsprechend geformten Öffnung 43 im Gehäuse 41 der Prüfvorrichtung 40 ein geringes Spiel auf. Eine Längsachse 58 der Positionierungseinrichtung 50 ist, wenn ein Endoskop 10 in die Positionierungseinrichtung 50 eingesetzt ist, parallel zu oder identisch mit einer Längsachse des Endoskops 10. Ein Kragen 54 an der Positionierungseinrichtung 50 liegt bei der vorgesehenen Position der Positionierungseinrichtung 50 an einer dafür vorgesehenen Fläche am Gehäuse 41 der Prüfvorrichtung 40 an und wird dort durch eine Kugel-Rasteinrichtung 46 gehalten. Dabei greift der in das Gehäuse 41 eingesetzte Stift 45 in einen Schlitz bzw. eine Nut 53 in der Positionierungseinrichtung 50 ein.

Somit stellen u. a. der Stift 74 am Referenzkörper 70 und die Nut 48 im Gehäuse 41, der Stift 45 am Gehäuse 41 und die Nut 53 in der Positionierungseinrichtung 50, der Kragen 54 an der Positionierungseinrichtung 50 und die Kugel-Rasteinrichtung 46 am Gehäuse 41 sowie der Anschlag 56 und die Kugel-Rasteinrichtung 57 in Zusammenwirkung mit einer in Figur 4 nicht dargestellten Ausnehmung im Endoskop 10 sicher, dass das distale Ende 12 des Endoskops 10 in einer vorbestimmten Position und Richtung relativ zu der Referenzoberfläche 72 in der Prüfvorrichtung 40 angeordnet ist.

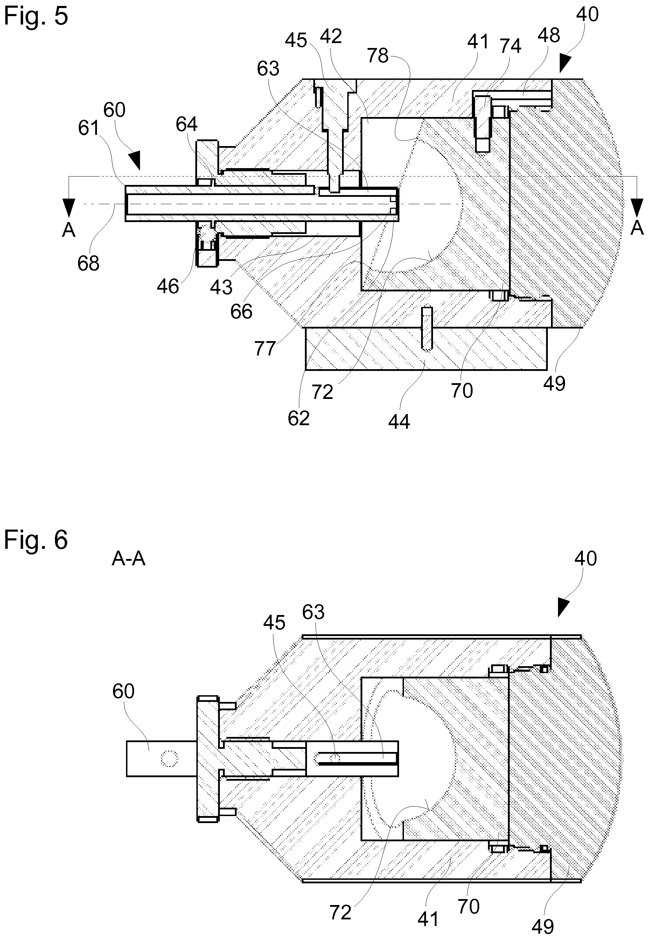

Die Figuren 5 und 6 zeigen schematische Darstellungen einer weiteren Prüfvorrichtung 40 in zwei verschiedenen Schnitten. Die in Figur 5 dargestellte Schnittebene entspricht der Schnittebene in Figur 4 rechts. Die in Figur 6 dargestellte Schnittebene A-A ist in Figur 5 eingezeichnet und steht senkrecht zur Schnittebene der Figur 5.

Die in den Figuren 5 und 6 dargestellte Prüfvorrichtung 40 unterscheidet sich von der oben dargestellten Prüfvorrichtung dadurch, dass eine andere Positionierungseinrichtung 60 in die Öffnung 43 im Gehäuse 41 eingesetzt ist. Das Gehäuse 41, der Hohlraum 42, die Öffnung 43, der Standfuß 44, der Stift 45, die Kugel-Rasteinrichtung 46, die Nut 48, der Schraubverschluss 49 und der Referenzkörper 70 entsprechen denen der oben anhand der Figuren 2 bis 4 dargestellten Prüfvorrichtung.

Die in den Figur 5 und 6 gezeigte Positionierungseinrichtung 60 ist wesentlich kürzer als die oben anhand der Figuren 2 und 4 dargestellte Positionierungseinrichtung 50. Das vom Hohlraum 42 im Gehäuse 41 abgewandte Ende 61 der Positionierungseinrichtung 60 ragt aus der Öffnung 43 nur wenig hervor. An dem in den Hohlraum 42 ragenden Ende 62 der im Wesentlichen rohrförmigen Positionierungseinrichtung 60 ist ein mechanischer Anschlag 66 in Form eines das Lumen der Positionierungseinrichtung 60 reduzierenden Kragens angeordnet. Die Positionierungseinrichtung 60 wird ähnlich wie die oben dargestellte Positionierungseinrichtung 50 durch Wechselwirkung eines Kragens 64 mit der Kugel-Rasteinrichtung 46 am Gehäuse 41 der Prüfeinrichtung 40 gehalten. Der Stift 45 am Gehäuse 41 greift in eine Nut 63 in der Positionierungseinrichtung 60 ein. Eine Längsachse 68 der Positionierungseinrichtung 60 ist, wenn ein Endoskop 10 in die Positionierungseinrichtung 60 eingesetzt ist, parallel zu oder identisch mit einer Längsachse des Endoskops 10.

Die in den Figuren 5 und 6 gezeigte Positionierungseinrichtung 60 ist für die Positionierung eines distalen Endes eines flexiblen Endoskops gegenüber der Referenzoberfläche 72 ausgebildet. In der Zusammenschau der Figuren 4 bis 6 ist erkennbar, dass die Positionierungseinrichtungen 50, 60 an der Prüfvorrichtung 40 gegeneinander ausgetauscht werden können. Dazu ist kein Werkzeug erforderlich, die Positionierungseinrichtungen 50, 60 müssen lediglich gegen die Rastkraft der Kugel-Rasteinrichtung 46 in das Gehäuse 41 der Prüfvorrichtung 40 geschoben oder aus diesem gezogen werden. Neben Positionierungseinrichtungen für starre Endoskope mit einer bestimmten Schaftlänge und einem bestimmten Schaftdurchmesser und für flexible Endoskope mit einem bestimmten Schaftdurchmesser können weitere Positionierungseinrichtungen für andere Schaftdurchmesser oder andere Schaftlängen in die Öffnung 43 des Gehäuses 41 eingesetzt werden.

Ein weiterer Aspekt der einfachen Entnehmbarkeit und Einsetzbarkeit der Positionierungseinrichtungen 50, 60 ist, dass die Prüfvorrichtung 40 einfach zerlegt und alle Bestandteile einschließlich der Positionierungseinrichtung 50, 60 leicht gereinigt und - beispielsweise durch Autoklavieren - sterilisiert werden können. Dazu trägt auch bei, dass nach Abnehmen des Schraubverschlusses 49 der Referenzkörper 70 aus dem Hohlraum 42 entnommen werden kann. Zum Zerlegen und zum Zusammensetzen der Prüfvorrichtung 40 ist kein Werkzeug erforderlich.

Ein besonderer Aspekt der in den Figuren 4 bis 6 gezeigten Prüfvorrichtung 40 ist, dass die Referenzoberfläche 72 einen Ausschnitt aus einer Kugeloberfläche bildet. Die Positionierungseinrichtungen 50, 60 sind so ausgebildet, dass das distale Ende 12 eines in die Positionierungseinrichtung 50; 60 eingesetzten Endoskops 10 am Mittelpunkt der Kugeloberfläche angeordnet ist und damit von allen Punkten der Referenzoberfläche 72 den gleichen Abstand hat. Einige Endoskope weisen ein gekrümmtes Objektfeld auf, d.h. die Menge der Orte, die gleichzeitig scharf (beispielsweise auf einen Bildsensor einer Kamera 31) abgebildet werden, bildet eine gekrümmte Fläche. Die Krümmung dieser Fläche ist insbesondere an die typische Krümmung einer Oberfläche eines Objekts, für deren Betrachtung das Endoskop vorgesehen ist, angepasst. Die Krümmung der Referenzoberfläche 72 und ihr Radius bzw. Abstand vom distalen Ende 12 des Endoskops 10 können an die Objektfeldkrümmung des Endoskops 10 angepasst sein, für das die Prüfvorrichtung 40 vorgesehen ist. Dies vereinfacht die Fokussierung des in die Prüfvorrichtung 40 eingesetzten Endoskops.

Ferner ist durch die kugelflächenförmige Gestalt der Referenzoberfläche 72 und die vorgesehene Position des distalen Endes 12 des Endoskops am Mittelpunkt der Kugeloberfläche der Abstand des Punkts auf der Referenzoberfläche 72, der in der Mitte des vom Endoskop 10 erzeugten Bilds liegt, vom distalen Ende 12 des Endoskops 10 unabhängig von der Blickrichtung des Endoskops. Die Prüfvorrichtung 40 kann somit für Endoskope mit unterschiedlichen Blickrichtungen verwendet werden.

In den Figuren 4 bis 6 ist ferner erkennbar, dass der Rand (in den Figur 4 und 5 insbesondere die in der Projektion auf die dargestellte Schnittebene gerade Linie zwischen den Punkten 77, 78) der Referenzoberfläche 72 nicht in einer Ebene senkrecht zur Längsachse 58, 68 der Positionierungseinrichtung 50; 60 liegt. Stattdessen liegt der Rand der Referenzoberfläche 72 in einer Ebene, deren Normale einen Winkel von ca. 30 Grad zur Längsachse 58, 68 der Positionierungseinrichtung 50; 60 bildet. Dies vergrößert das Spektrum der möglichen Blickrichtungen von Endoskopen, für die die Prüfvorrichtung 40 verwendbar ist. Beispielsweise ist bei diesem Winkel die Prüfvorrichtung auch für ein Endoskop verwendbar, dessen Blickrichtung einen Winkel von 90 Grad zu seiner Längsachse bildet, und dessen Sichtfeld 60 Grad breit ist. Durch noch größere Winkel zwischen der Normale der Ebene, in der der Rand der Referenzoberfläche 72 liegt, und der Längsachse 58, 68 der Positionierungseinrichtung 50, 60 kann das Spektrum der möglichen Blickrichtungen der zu prüfenden Endoskope weiter vergrößert werden. Gleichzeitig ist die halbkugelförmige Referenzoberfläche 72 leicht herstellbar, beispielsweise durch Gießen oder mittels spanabhebender Verfahren, ohne dass Hinterschneidungen geschaffen werden müssten.

Wichtig für die genannten Vorteile der gekippten Anordnung der Referenzoberfläche 72 ist vor allem, dass die Gerade durch gegenüberliegende Punkte bzw. Orte 77, 78 am Rand der Referenzoberfläche 72 gegenüber der Längsachse 58, 68 der Positionierungseinrichtung 50, 60 gekippt ist. Die Gestalt des Rands der Referenzoberfläche 72 zwischen diesen beiden Punkten 77, 78 kann von einem ebenen Kreisring abweichen, ohne die genannten Vorteile zu schmälern. In Verallgemeinerung dieser Überlegung kann die Referenzoberfläche 72 statt der Gestalt eines Ausschnitts einer Kugeloberfläche die Gestalt eines Ausschnitts einer Kreiszylinderoberfläche aufweisen. In diesem Fall liegt die Zylinderachse der Kreiszylinderfläche insbesondere senkrecht zu den Zeichenebenen der Figuren 4 und 5. Die Gerade zwischen den Punkten 77, 78 stellt dann nicht mehr den Rand der Referenzoberfläche 72, sondern eine Verbindungsgerade der Punkte 77, 78 dar, die zur Längsachse 58, 68 der Positionierungseinrichtung 50, 60 einen Winkel von beispielsweise 60 Grad bildet.

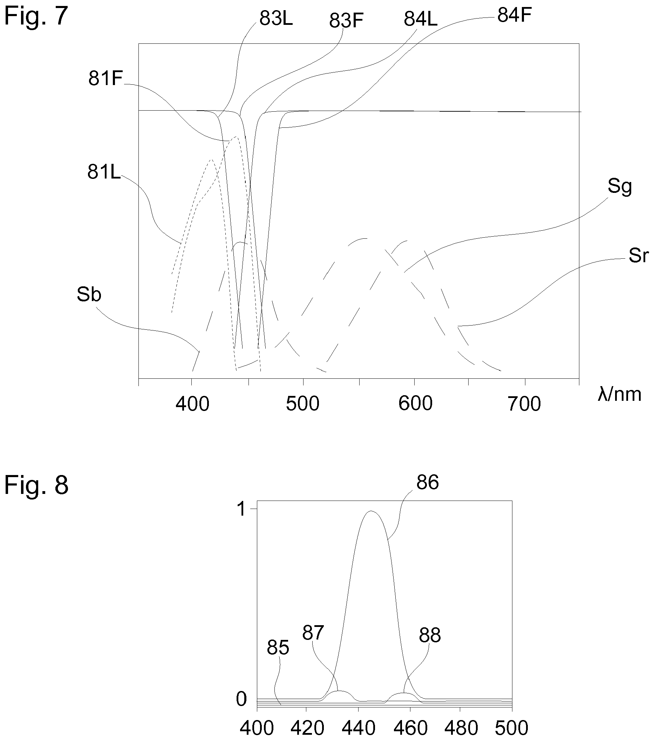

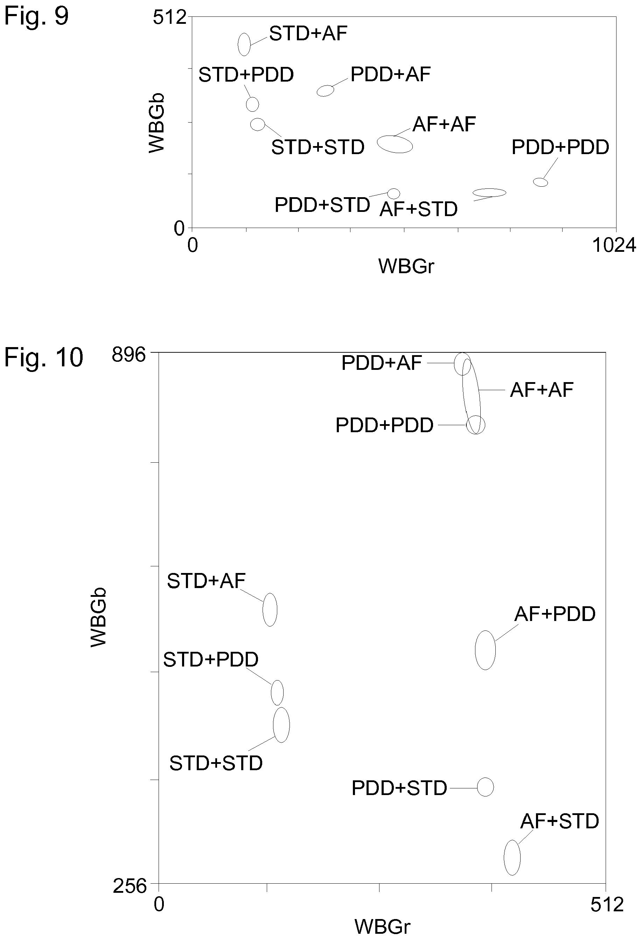

Nachfolgend werden anhand der Figuren 7 bis 10 Verfahren zum Prüfen eines optischen Untersuchungssystems beschrieben, die mittels der oben dargestellten Prüfvorrichtung 40 ausgeführt werden können. Zunächst werden anhand der Figuren 7 und 8 Spektren und anhand der Figuren 9 und 10 Weißabgleich-Parameter dargestellt, auf Grundlage derer eine Funktionsfähigkeit oder eine andere Eigenschaft des optischen Untersuchungssystems bestimmt werden kann.

Figur 7 zeigt eine schematische Darstellung von Fluoreszenz-Anregungsspektren sowie Transmissionsspektren von Beleuchtungs- und Beobachtungsfiltern, die für verschiedene Arten der Fluoreszenz-Diagnostik verwendet werden. Der Abszisse ist die Wellenlänge λ zugeordnet. Gezeigt sind neben dem Fluoreszenz-Anregungsspektrum 81L, dem Transmissionsspektrum 83L des Beleuchtungsfilters und dem Transmissionsspektrum 84L des Beobachtungsfilters für PDD auch das Fluoreszenz-Anregungsspektrum 81F, das Transmissionsspektrum 83F des Beleuchtungsfilters und das Transmissionsspektrum 84F des Beobachtungsfilters zur Beobachtung von Autofluoreszenz (AF) von Gewebe.

In Figur 7 sind ferner die spektralen Empfindlichkeiten Sb, Sg, Sr der blauen, grünen und roten Farbrezeptoren des menschlichen Auges dargestellt. Da Kameras möglichst weitgehend an das Farbempfinden des menschlichen Auges angepasst werden, weisen sie in der Regel ähnliche spektrale Empfindlichkeiten auf. Im Vergleich der Transmissionsspektren 83L, 83F, 84L, 84F der Beleuchtungs- und Beobachtungsfilter für PDD und AF mit den spektralen Empfindlichkeiten der Farbrezeptoren des menschlichen Auges wird deutlich, dass die geringen Unterschiede zwischen den Transmissionsspektren der Beleuchtungs- und Beobachtungsfilter für PDD und AF für das menschliche Auge nur unter guten Bedingungen im unmittelbaren Vergleich - der selten möglich ist - erkennbar sind.

Figur 8 zeigt eine schematische Darstellung verschiedener Produkte jeweils eines Transmissionsspektrums eines Beleuchtungsfilters und eines Transmissionsspektrums eines Beobachtungsfilters. Die Kurven sind vertikal geringfügig gegeneinander verschoben, damit sie leichter unterschieden werden können. Tatsächlich sind alle Produkte bei Wellenlängen um 400 nm und bei Wellenlängen um 500 nm näherungsweise 0.

Das Produkt 85 aus dem Transmissionsspektrum 83L des PDD-Beleuchtungsfilters und dem Transmissionsspektrum 84F des AF-Beobachtungsfilters ist für alle Wellenlängen sehr klein bzw. näherungsweise 0. Das AF-Beobachtungsfilter ist somit für remittiertes PDD-Anregungslicht nicht transparent.

Das Produkt 86 aus dem Transmissionsspektrum 83F des Beleuchtungsfilters für AF-Diagnostik und dem Transmissionsspektrum 84L des Beobachtungsfilters für PDD ist für Wellenlängen im Bereich von ca. 430 nm bis ca. 460 nm deutlich größer als 0. Das PDD-Beobachtungsfilter ist für remittiertes AF-Anregungslicht somit in einem deutlich sichtbaren Maß transparent.

Das Produkt 87 aus dem Transmissionsspektrum 83L des Beleuchtungsfilters für PDD und dem Transmissionsspektrum 84L des Beobachtungsfilters für PDD ist in einem kleinen Wellenlängenbereich zwischen ca. 430 nm und ca. 440 nm nicht 0. Das PDD-Beobachtungsfilter ist für remittiertes PDD-Anregungslicht geringfügig transparent.

Das Produkt 88 aus dem Transmissionsspektrum 83F des Beleuchtungsfilters für AF und dem Transmissionsspektrum 84F des Beobachtungsfilters für AF ist in einem kleinen Wellenlängenbereich in der Gegend von 460 nm nicht 0. Das AF-Beobachtungsfilter ist für remittiertes AF-Anregungslicht geringfügig transparent.

Bei Betrachtung einer weißen, nicht fluoreszierenden Referenzoberfläche mit einem optischen Untersuchungssystem kann somit unter günstigen Umständen unterscheidbar sein, ob ein PDD-Beleuchtungsfilter mit einem AF-Beobachtungsfilter oder ein AF-Beleuchtungsfilter mit einem PDD-Beobachtungsfilter kombiniert ist. Im ersten Fall wird ein extrem dunkles Bild beobachtet, im zweiten Fall ein im Vergleich zu korrekten Kombinationen von Beleuchtungsfilter und Beobachtungsfilter zu helles Bild. Kaum unterscheidbar hingegen ist, ob ein Beleuchtungsfilter für PDD mit einem Beobachtungsfilter für PDD oder ein Beleuchtungsfilter für AF mit einem Beobachtungsfilter für AF kombiniert ist. In beiden Fällen ist das Bild näherungsweise gleich hell, der Unterschied in der Wellenlänge ist für das menschliche Auge allenfalls unter sehr guten Bedingungen im unmittelbaren Vergleich unterscheidbar.

Figur 9 zeigt in einem schematischen Diagramm typische Weißabgleich-Parameter nach einem Weißabgleich an einer weißen, nicht fluoreszierenden Referenzoberfläche bei verschiedenen Kombinationen von Beleuchtungsfiltern und Beobachtungsfiltern. Die Referenzoberfläche ist in diesem Beispiel eine Oberfläche eines Referenzkörpers aus weißem PTFE. Der Abszisse ist der Weißabgleich-Parameter WBGr, der Ordinate der Weißabgleich-Parameter WBGb zugeordnet, wobei der dritte Weißabgleich-Parameter WBGg per Konvention den Wert WBGg = 128 = 0x080 aufweist. Bei den Messpunkten ist die Filterkombination jeweils angegeben, wobei die Angabe vor dem Pluszeichen das Beleuchtungsfilter und die Angabe nach dem Pluszeichen das Beobachtungsfilter angibt. Dabei bedeutet "STD" kein Filter (Weißlicht), "PDD" ein Filter für PDD und "AF" ein Filter für AF. Zulässige Filterkombinationen sind STD+STD, PDD+PDD und AF+AF.

Es ist erkennbar, dass verschiedene Filterkombinationen verschiedene Weißabgleich-Parameter zur Folge haben, die eindeutig zugeordnet und unterschieden werden können. Nach Durchführung eines Weißabgleichs mit einer geeigneten Referenzoberfläche kann somit aus dem Weißabgleich-Parameter auf die vorliegende Filterkombination geschlossen werden.

Da die Weißabgleich-Parameter von Kameratyp zu Kameratyp und in manchen Fällen sogar von Kamera zu Kamera variieren, können die bei einem Weißabgleich gewonnen Weißabgleich-Parameter beispielsweise durch in der Kamera abgelegte Korrektur-Parameter korrigiert werden. Die in der Kamera abgelegten Korrektur-Parameter sind beispielsweise ohne Beleuchtungs- und Beobachtungsfilter an einer weißen Fläche gewonnene Weißabgleich-Parameter. Diese Korrektur-Parameter können statt in der Kamera 31 auch in der Kamerasteuerung 35 abgelegt sein. Weitere Korrekturen können für die Bauform oder den Typ des Endoskops erfolgen, da starre und flexible Endoskope, Endoskope mit unterschiedlichen Durchmessern oder für unterschiedliche Anwendungen unterschiedliche Transmissionsspektren im Beleuchtungs-Strahlengang und im Beobachtungs-Strahlengang aufweisen.

Bei Verwendung einer nicht-weißen Referenzoberfläche für einen Weißabgleich kann die Genauigkeit bzw. Zuverlässigkeit der Unterscheidung verschiedener Filterkombinationen anhand der Weißabgleich-Parameter weiter verbessert werden. Dies gilt insbesondere, wenn Kanten bzw. Flanken in Absorptions- oder Fluoreszenzanregungsspektren in der Nähe der Filterkanten der zu unterscheidenden Beleuchtungs- und Beobachtungsfilter liegen.

Figur 10 zeigt in einem schematischen Diagramm Weißabgleich-Parameter für verschiedene Filterkombinationen bei einem Weißabgleich an einer Referenzoberfläche aus einer deckenden Schicht der von Marabu vertriebenen Farbe Maragloss GO 320 Fluoresco-Gelb. Abszisse und Ordinate sowie Kennzeichnung der Messwerte entsprechen denen aus Figur 9. Ein Vergleich der Figuren 9 und 10 zeigt, dass eine besonders sichere Identifikation der vorliegenden Filterkombination möglich ist, wenn sowohl die Weißabgleich-Parameter aus einem Weißabgleich an einer Teflonoberfläche als auch die Weißabgleich-Parameter aus einem Weißabgleich an einer deckenden Schicht von Marabu Maragloss GO Fluoresco 320 gelb kombiniert werden. Weitere Verbesserungen sind beispielsweise durch eine logische oder algebraische Verknüpfung der Weißabgleich-Parameter möglich.

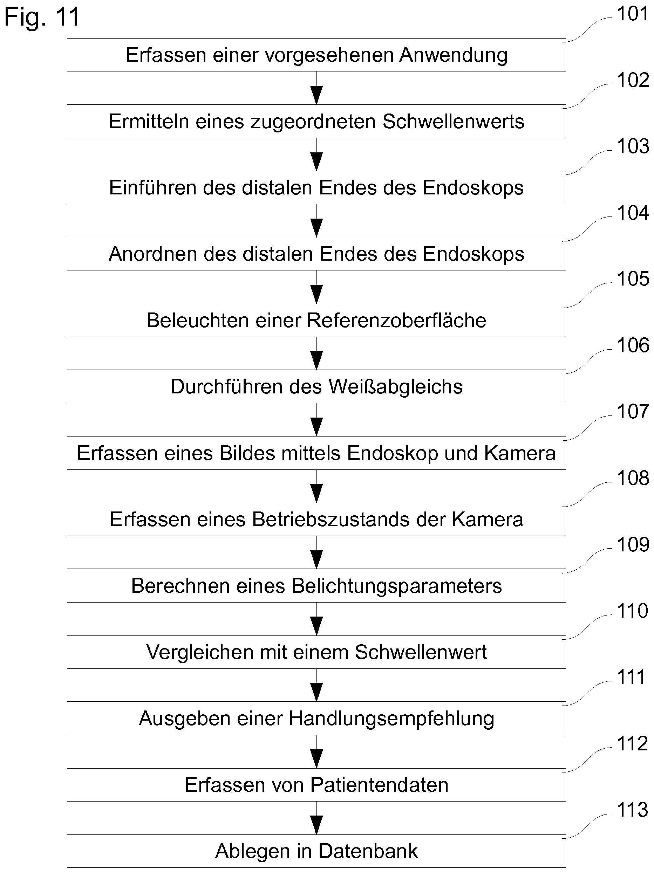

Figur 11 zeigt ein schematisches Flussdiagramm eines Verfahrens zum Prüfen eines optischen Untersuchungssystems mit einer bildgebenden Einrichtung, einer Kamera und einer Lichtquelle zur optischen Untersuchung eines Objekts. Obwohl das Verfahren auch an optischen Untersuchungssystemen und mit Prüfvorrichtungen anwendbar ist, die sich von den oben dargestellten unterscheiden, werden nachfolgend zur Vereinfachung des Verständnisses beispielhaft Bezugszeichen aus den Figuren 1 bis 6 verwendet. Bei dem Verfahren werden Merkmale von Spektren, wie sie in den Figuren 7 und 8 dargestellt sind, und von Weißabgleich-Parametern, wie sie in den Figuren 9 und 10 dargestellt sind, verwendet, um die Funktionsfähigkeit des optischen Untersuchungssystems zu prüfen oder eine andere Eigenschaft des optischen Untersuchungssystems zu bestimmen, beispielsweise darin enthaltene Beleuchtungs- und Beobachtungsfilter.

Bei einem optionalen ersten Schritt wird eine vorgesehene Anwendung des optischen Untersuchungssystems erfasst, beispielsweise an einer Benutzerschnittstelle nach einer entsprechenden Aufforderung. Bei einem optionalen zweiten Schritt wird eine der vorgesehenen Anwendung zugeordnete Bedingung an einen Betriebszustand der Kamera 31 des optischen Untersuchungssystems ermittelt, beispielsweise durch Auslesen einer Look-up-Tabelle. Eine oder mehrere Bedingungen für den Betriebszustand der Kamera 31 können alternativ unveränderlich vorgegeben sein.

Bei einem dritten Schritt 103 wird ein distales Ende einer bildgebenden Einrichtung, insbesondere eines Endoskops 10, durch eine Öffnung 43 in einen Hohlraum 42 in einem lichtdichten Gehäuse 41 eingeführt. Bei einem optionalen vierten Schritt 104, der nach dem dritten Schritt 103 oder gleichzeitig mit diesem ausgeführt werden kann, wird das distale Ende der bildgebenden Einrichtung in einer vorbestimmten Position und Richtung relativ zu einer in dem Hohlraum 42 angeordneten Referenzoberfläche 72 angeordnet. Dies erfolgt unterstützt durch eine Positionierungseinrichtung, die die bildgebende Einrichtung 10, insbesondere deren distales Ende 12 führt und/oder form- oder kraftschlüssig hält.

Bei einem fünften Schritt 105 wird die Referenzoberfläche 72 mit Beleuchtungslicht mit einem Beleuchtungsspektrum beleuchtet. Wenn die bildgebende Einrichtung ein Endoskop 10 ist, erfolgt die Beleuchtung insbesondere mittels des Endoskops bzw. über einen Beleuchtungs-Strahlengang im Endoskop 10. Bei einem optionalen sechsten Schritt 106 wird ein Weißabgleich, wie er oben beschrieben ist, durchgeführt während die Referenzoberfläche beleuchtet wird. Dabei werden beispielsweise Weißabgleich-Parameter WBGr, WBGb eingestellt.

Bei einem siebten Schritt 107 wird während des Beleuchtens der Referenzoberfläche 72 mittels der bildgebenden Einrichtung 10 und der Kamera 31 ein Bild erfasst. Bei einem achten Schritt 108 wird der während des siebten Schritts 107 vorliegende Betriebszustand der Kamera 31 erfasst, insbesondere aus der Kamera 31 oder der Kamerasteuerung 35 ausgelesen. Alternativ wird beispielsweise ein Rauschpegel oder ein Signal-Rausch-Abstand im erfassten Bild bestimmt, aus dem auf den Betriebszustand der Kamera 31 geschlossen wird. Der Betriebszustand der Kamera umfasst beispielsweise Weißabgleich-Parameter WBGr, WBGb, eine für alle Farbkanäle geltende Belichtungszeit, eine für alle Farbkanäle geltende Verstärkung oder für einzelne Farbkanäle geltende Belichtungszeiten und Verstärkungen.

Bei einem optionalen neunten Schritt 109 wird aus dem erfassten Betriebszustand der Kamera ein Belichtungsparameter ermittelt, insbesondere berechnet. Beispielsweise wird aus einer Belichtungszeit T und einer Verstärkung G ein Belichtungsparameter E nach der Formel E=a·T^{b}·G^{c} berechnet. Bei einem ebenfalls optionalen zehnten Schritt 110 wird der ermittelte Belichtungsparameter mit einem oder mehreren einer vorgesehenen Anwendung zugeordneten oder pauschal geltenden Schwellenwerten verglichen. Alternativ oder zusätzlich wird der Betriebszustand der Kamera bzw. die ihn charakterisierenden Parameter mit anderen der vorbestimmten Anwendung des optischen Untersuchungssystems zugeordneten und pauschal geltenden Bedingungen verglichen. Das Ergebnis des Vergleichs zeigt die Funktionsfähigkeit oder eine andere Eigenschaft des optischen Untersuchungssystems an.

Wenn beim achten Schritt 108 Weißabgleich-Parameter erfasst werden, kann anhand eines Vergleichs der Weißabgleich-Parameter mit Schwellenwerten bestimmt werden, welches Beleuchtungsfilter bzw. welches Beleuchtungs-Spektrum und welches Transmissionsspektrum im Beobachtungs-Strahlengang beim Erfassen des Bilds vorlag. Dazu liegen die Schwellenwerte insbesondere zwischen den beispielsweise in den Figuren 9 und 10 erkennbaren Bereichen, die den verschiedenen Filterkombinationen zugeordnet sind.

Wenn beim achten Schritt 108 Belichtungszeiten, Verstärkungen oder andere Parameter erfasst werden, die einzelnen Farbkanälen zugeordnet sind, sind diese Parameter des Betriebszustands ähnlich wie Weißabgleich-Parameter von der vorliegenden Filterkombination abhängig. Aus den den einzelnen Farbkanälen zugeordneten Parametern des Betriebszustands kann deshalb ebenfalls auf die vorliegende Filterkombination geschlossen werden. Dazu werden die den einzelnen Farbkanälen zugeordneten Parameter des Betriebszustands oder aus diesen berechnete Belichtungsparameter mit entsprechenden Schwellenwerten verglichen.

Bei einem optionalen elften Schritt 111 wird eine Meldung ausgegeben, die eine Aussage über die Funktionsfähigkeit des optischen Untersuchungssystems, eine Handlungsempfehlung und/oder eine Handlungsanweisung umfassen kann. Bei einem zwölften Schritt 112, der auch zu einem beliebigen anderen Zeitpunkt in dem Verfahren ausgeführt werden kann, werden Patientendaten erfasst, beispielsweise mittels einer Benutzerschnittstelle. Bei einem optionalen dreizehnten Schritt 113 werden die Patientendaten, das Ergebnis des Prüfverfahrens hinsichtlich der Funktionsfähigkeit oder hinsichtlich der anderen Eigenschaft des optischen Untersuchungssystems und optional das Ergebnis einer nachfolgenden oder vorangehenden Untersuchung eines Patienten mittels des optischen Untersuchungssystems in einer Datenbank abgelegt.

Insbesondere bei Verwendung einer Kamera 31 können ferner Modellbezeichnungen, Seriennummern, Software- oder Firmware-Versionen und andere Daten von Komponenten des optischen Untersuchungssystems über eine Kommunikationsleitung 39 abgefragt und zur Dokumentation bzw. Protokollierung in der Datenbank abgelegt werden. Ferner kann in der Datenbank oder separat auf einem anderen Datenträger die Untersuchung des Patienten dokumentiert bzw. protokolliert werden. Dabei werden beispielsweise Bilder bzw. ein Videodatenstrom der Kamera 31 in der Datenbank (beispielsweise in einem MPEG-Format) oder auf einem Videoband abgelegt.

### Bezugszeichen

- 10: Endoskop
- 11: proximales Ende des Endoskops 10
- 12: distales Ende des Endoskops 10
- 13: Beobachtungsfilter des Endoskops 10
- 14: Okular am proximalen Ende des Endoskops 10
- 15: Kupplung am Endoskop 10 für Lichtleitkabel 19
- 17: Schaft des Endoskops 10
- 19: Lichtleitkabel zu Kopplung des Endoskops 10 mit der Lichtquellenvorrichtung 20

- 20: Lichtquellenvorrichtung
- 22: Lichtquelle der Lichtquellenvorrichtung 20
- 23: erste Sammellinse
- 24: Beleuchtungsfilter der Lichtquellenvorrichtung 20
- 25: zweite Sammellinse
- 26: Kupplung an der Lichtquellenvorrichtung 20 für Lichtleitkabel 19

- 31: Kamera
- 33: Signalkabel
- 35: Kamerasteuerung
- 37: Bildschirm
- 39: Kommunikationsleitung

- 40: Prüfvorrichtung
- 41: lichtdichtes Gehäuse der Prüfvorrichtung 40
- 42: Hohlraum im Gehäuse 41
- 43: Öffnung im Gehäuse 41
- 44: Standfuß des Gehäuses 41
- 45: Stift
- 46: Kugel-Rasteinrichtung
- 48: Nut im Gehäuse 41
- 49: Schraubverschluss

- 50: erste Positionierungseinrichtung
- 51: vom Hohlraum 42 abgewandtes Ende der ersten Positionierungseinrichtung 50
- 52: dem Hohlraum 42 zugewandtes Ende der ersten Positionierungseinrichtung 50
- 53: Nut in der ersten Positionierungseinrichtung 50
- 56: Anschlag für Endoskop 10
- 57: Kugel-Rasteinrichtung am vom Hohlraum 42 abgewandten Ende 51 der ersten Positionierungseinrichtung
- 58: Längsachse der ersten Positionierungseinrichtung 50

- 60: zweite Positionierungseinrichtung
- 61: vom Hohlraum 42 abgewandtes Ende der zweiten Positionierungseinrichtung 60
- 62: dem Hohlraum 42 zugewandtes Ende der zweiten Positionierungseinrichtung 60
- 63: Nut in der zweiten Positionierungseinrichtung 60
- 66: Anschlag für distales Ende 12 des Endoskops 10
- 68: Längsachse der zweiten Positionierungseinrichtung 60

- 70: Referenzkörper der Prüfvorrichtung 40
- 72: Referenzoberfläche am Referenzkörper 70
- 74: Stift am Referenzkörper 70
- 75: Indikatorbereich an der Referenzfläche 72
- 76: Referenzbereich an der Referenzfläche 72
- 77: erster Ort am Rand der Referenzoberfläche 72
- 78: zweiter Ort am Rand der Referenzoberfläche 72
- 81: Fluoreszenz-Anregungsspektrum
- 82: Fluoreszenz-Abregungsspektrum
- 83: Transmissionsspektrum des Beleuchtungsfilters
- 83L: Transmissionsspektrum des Beleuchtungsfilters für PDD
- 83F: Transmissionsspektrum des Beleuchtungsfilters für AF
- 84: Transmissionsspektrum des Beobachtungsfilters
- 84L: Transmissionsspektrum des Beobachtungsfilters für PDD
- 84F: Transmissionsspektrum des Beobachtungsfilters für AF
- 85: Produkt aus 83L und 84F
- 86: Produkt aus 83F und 84L
- 87: Produkt aus 83L und 84L
- 88: Produkt aus 83F und 84F

- 101: erster Schritt (Erfassen einer vorgesehenen Anwendung)
- 102: zweiter Schritt (Ermitteln eines Schwellenwerts)
- 103: dritter Schritt (Einführen des distalen Endes des Endoskops)
- 104: vierter Schritt (Anordnen des distalen Endes des Endoskops)
- 105: fünfter Schritt (Beleuchten einer Referenzoberfläche)
- 106: sechster Schritt (Durchführen eines Weißabgleichs)
- 107: siebter Schritt (Erfassen eines Bilds mittels Endoskop und Kamera)
- 108: achter Schritt (Erfassen eines Betriebszustands der Kamera)
- 109: neunter Schritt (Berechnen eines Belichtungsparameters)
- 110: zehnter Schritt (Vergleichen mit einem Schwellenwert)
- 111: elfter Schritt (Ausgeben einer Handlungsempfehlung)
- 112: zwölfter Schritt (Erfassen von Patientendaten)
- 113: dreizehnter Schritt (Ablegen in Datenbank)

## Patentansprüche

1. **Prüfvorrichtung** (40) für ein optisches Untersuchungssystems mit einer bildgebenden Einrichtung (10) und einer Lichtquelle (22) zur optischen Untersuchung eines Objekts in remittiertem Licht und/oder Fluoreszenzlicht, mit:
einem **Gehäuse** (41) mit einem Hohlraum (42) und einer Öffnung (43) zum Einführen eines distalen Endes (12) der bildgebenden Einrichtung (10) in den Hohlraum (43);
einer **Referenzoberfläche** (72) mit vorbestimmten optischen Eigenschaften in dem Hohlraum (42), zumindest entweder zur Remission von auf die Referenzoberfläche (72) gerichtetem Beleuchtungslicht oder zur Emission von Fluoreszenzlicht;
einer im Wesentlichen rohrförmigen **Positionierungseinrichtung** (50; 60) zum Halten der bildgebenden Einrichtung (10) bei einer durch die Positionierungseinrichtung (50; 60) vorbestimmten Position des distalen Endes (12) der bildgebenden Einrichtung (10) relativ zur Referenzfläche (72),
wobei die vorbestimmte Position des distalen Endes (12) der bildgebenden Einrichtung (10) innerhalb des Hohlraums (42) ist,
wobei die Positionierungseinrichtung (50) an ihrem vom Hohlraum (42) abgewandten Ende (51) einen mechanischen Anschlag (56) oder eine Kugel-Rasteinrichtung (57) zum Halten des proximalen Endes (11) einer in die Prüfvorrichtung (40) eingesetzten bildgebenden Einrichtung (10) aufweist
oder wobei die Positionierungseinrichtung (60) an einem dem Hohlraum (42) zugewandten Ende (62) einen mechanischen **Anschlag** (66) aufweist, der so ausgebildet ist, dass das distale Ende (12) einer in die Prüfvorrichtung (40) eingesetzten bildgebenden Einrichtung (10) an der vorbestimmten Position liegt, wenn die bildgebende Einrichtung (10) an dem Anschlag (66) anliegt.

2. Prüfvorrichtung (40) nach einem der vorangehenden Ansprüche, bei der die Positionierungseinrichtung (50; 60) ferner dazu ausgebildet ist, eine **Rotation** einer in die Prüfvorrichtung (40) eingesetzten bildgebenden Einrichtung (10) um ihre Längsachse zu unterbinden.

3. Prüfvorrichtung (40) nach einem der vorangehenden Ansprüche, bei der die Prüfvorrichtung (40) so ausgebildet ist, dass die Positionierungseinrichtung (50; 60) ohne Verwendung von Werkzeug von dem Gehäuse (41) getrennt und wieder mit ihm verbunden werden kann.

4. Prüfvorrichtung (40) nach dem vorangehenden Anspruch, ferner mit:
Einrichtungen (45, 53; 63) am Gehäuse (41) und an der Positionierungseinrichtung (50; 60) zum formschlüssigen Unterbinden einer Rotation der Positionierungseinrichtung (50; 60) um ihre Längsachse (58; 68).

5. Prüfvorrichtung (40) nach Anspruch 3 oder 4, ferner mit:
einer Rasteinrichtung (46), die dazu ausgebildet ist, die Positionierungseinrichtung (50; 60) an der Öffnung (43) lösbar zu befestigen.

6. Prüfvorrichtung (40) nach einem der vorangehenden Ansprüche, bei der die Referenzoberfläche (72)
einen Ausschnitt aus einer **Kugeloberfläche** bildet, wobei die für das distale Ende (12) der bildgebenden Einrichtung (10) vorgesehene Position am Mittelpunkt der Kugeloberfläche liegt; oder
einen Ausschnitt aus einer **Kreiszylinderfläche** bildet, wobei die für das distale Ende (12) der bildgebenden Einrichtung (10) vorgesehene Position an der Symmetrieachse der Kreiszylinderfläche liegt.

7. Prüfvorrichtung (40) nach dem vorangehenden Anspruch, bei der
die Referenzoberfläche (72) einen Ausschnitt aus einer Kugeloberfläche bildet, wobei zwei gegenüberliegende Orte (77, 78) am Rand der Referenzoberfläche (72) auf einer Geraden (79) liegen, die einen **Winkel** im Bereich von 40 Grad bis 80 Grad zur Längsachse einer von der Positionierungseinrichtung (50; 60) gehaltenen bildgebenden Einrichtung (10) bildet; oder
die Referenzoberfläche (72) einen Ausschnitt aus einer Kugeloberfläche bildet, wobei der Rand der Referenzoberfläche (72) in einer Ebene (78) liegt, deren Normale einen **Winkel** im Bereich von 10 Grad bis 50 Grad zur Längsachse einer von der Positionierungseinrichtung (50; 60) gehaltenen bildgebenden Einrichtung (10) bildet; oder
die Referenzoberfläche (72) einen Ausschnitt aus einer Kreiszylinderfläche bildet, wobei eine Gerade (79) durch zwei gegenüberliegende Ränder (77, 78) der Referenzoberfläche (72) einen Winkel zwischen 40 Grad und 80 Grad zur Längsachse einer von der Positionierungseinrichtung (50; 60) gehaltenen bildgebenden Einrichtung (10) bildet.

8. Prüfvorrichtung (40) nach einem der vorangehenden Ansprüche, wobei die Prüfvorrichtung **zerlegbar** und **sterilisierbar** ist.

9. Prüfvorrichtung (40) nach einem der vorangehenden Ansprüche, bei der die Referenzoberfläche (72) **weiß** ist oder einen weißen Bereich umfasst.

10. Prüfvorrichtung (40) nach einem der vorangehenden Ansprüche, bei welcher die Referenzoberfläche (72) durch **Polytetrafluorethylen** oder **Silikon** gebildet wird.

11. **Verfahren zum Prüfen** eines optischen Untersuchungssystems mit einer bildgebenden Einrichtung (10) und einer Lichtquelle (22) zur optischen Untersuchung eines Objekts in remittiertem Licht und Fluoreszenzlicht, mit folgenden Schritten:
Einführen (103) des distalen Endes (12) der bildgebenden Einrichtung (10) durch eine Öffnung (43) in einen Hohlraum (42) in einem **Gehäuse** (41);
Anordnen (104) des distalen Endes (12) der bildgebenden Einrichtung (10) an einer durch eine im Wesentlichen rohrförmigen Positionierungseinrichtung (50; 60) vor**bestimmten Position** relativ zu einer Referenzoberfläche (72) mit vorbestimmten optischen Eigenschaften in dem Hohlraum (42), wobei die vorbestimmte Position des distalen Endes (12) der bildgebenden Einrichtung (10) innerhalb des Hohlraums (42) ist und die Positionierungseinrichtung (50) an ihrem vom Hohlraum (42) abgewandten Ende (51) einen mechanischen Anschlag (56) oder eine Kugel-Rasteinrichtung (57) zum Halten des proximalen Endes (11) einer in die Prüfvorrichtung (40) eingesetzten bildgebenden Einrichtung (10) aufweist oder wobei die Positionierungseinrichtung (60) an einem dem Hohlraum (42) zugewandten Ende (62) einen mechanischen Anschlag (66) aufweist, der so ausgebildet ist, dass das distale Ende (12) einer in die Prüfvorrichtung (40) eingesetzten bildgebenden Einrichtung (10) an der vorbestimmten Position liegt, wenn die bildgebende Einrichtung (10) an dem Anschlag (66) anliegt;
**Beleuchten** (105) der Referenzoberfläche (72) mit Beleuchtungslicht mit einem vorbestimmten Spektrum mittels der bildgebenden Einrichtung (10);
**Erfassen** (107) von durch die Referenzoberfläche (72) remittiertem Licht mittels der bildgebenden Einrichtung (10);
Bestimmen der **Funktionsfähigkeit** oder einer anderen Eigenschaft des optischen Untersuchungssystems anhand des erfassten remittierten Lichts.

## Claims

1. Test apparatus (40) for an optical examination system having an imaging device (10) and a light source (22) for the optical examination of an object in reflected light and/or fluorescence light, comprising:
a housing (41) with a cavity (42) and an opening (43) for inserting a distal end (12) of the imaging device (10) into the cavity (43);
a reference surface (72) with predetermined optical properties in the cavity (42), at least either for diffusely reflecting illumination light directed onto the reference surface (72) or for emitting fluorescence light;
a substantially tubular positioning device (50; 60) for holding the imaging device (10) at a position of the distal end (12) of the imaging device (10) relative to the reference face (72), said position being predetermined by the positioning device (50; 60),
wherein the predetermined position of the distal end (12) of the imaging device (10) is within the cavity (42),
wherein, at its end (51) distant from the cavity (42), the positioning device (50) has a mechanical stop (56) or a ball-locking device (57) for holding the proximal end (11) of an imaging device (10) that has been inserted into the test device (40),
or wherein, at its end (62) facing the cavity (42), the positioning device (60) has a mechanical stop (66) that is embodied in such a way that the distal end (12) of an imaging device (10) that has been inserted into the test apparatus (40) lies at the predetermined position when the imaging device (10) rests against the stop (66).

2. Test apparatus (40) according to any one of the preceding claims, wherein the positioning device (50; 60) is further embodied to suppress a rotation of an imaging device (10) about its longitudinal axis, said imaging device having been inserted into the test apparatus (40).

3. Test apparatus (40) according to either of the preceding claims, wherein the test apparatus (40) is embodied in such a way that the positioning device (50; 60) can be separated from the housing (41) and reconnected therewith without the use of tools.

4. Test apparatus (40) according to the preceding claim, further comprising:
devices (45, 53; 63) at the housing (41) and at the positioning device (50; 60) for interlocking suppression of a rotation of the positioning device (50; 60) about its longitudinal axis (58; 68) .

5. Test apparatus (40) according to Claim 3 or 4, further comprising:
a latching device (46) that is embodied to detachably fasten the positioning device (50; 60) at the opening (43).

6. Test apparatus (40) according to any one of the preceding claims, wherein the reference surface (72)
forms a section of a spherical surface, wherein the position provided for the distal end (12) of the imaging device (10) lies at the centre point of the spherical surface; or
forms a section of a circular cylindrical face, wherein the position provided for the distal end (12) of the imaging device (10) lies on the axis of symmetry of the circular cylindrical face.

7. Test apparatus (40) according to the preceding claim, wherein
the reference surface (72) forms a section of a spherical surface, wherein two opposite locations (77, 78) lie at the edge of the reference surface (72) on a straight line (79) that forms an angle in the range from 40 degrees to 80 degrees in relation to the longitudinal axis of an imaging device (10) that is held by the positioning device (50; 60); or
the reference surface (72) forms a section of a spherical surface, wherein the edge of the reference surface (72) lies in a plane (78), the normal of which forms an angle in the range from 10 degrees to 50 degrees in relation to the longitudinal axis of an imaging device (10) that is held by the positioning device (50; 60); or
the reference surface (72) forms a section of a circular cylindrical face, wherein a straight line (79) through two opposite edges (77, 78) of the reference surface (72) forms an angle of between 40 degrees and 80 degrees in relation to the longitudinal axis of an imaging device (10) that is held by the positioning device (50; 60).

8. Test apparatus (40) according to any one of the preceding claims, wherein the test apparatus is disassemblable and sterilizable.

9. Test apparatus (40) according to any one of the preceding claims, wherein the reference surface (72) is white or comprises a white region.

10. Test apparatus (40) according to any one of the preceding claims, wherein the reference surface (72) is formed by polytetrafluoroethylene or silicone.

11. Method for testing an optical examination system with an imaging device (10) and a light source (22) for optical examination of an object in reflected light and fluorescence light, including the following steps:
inserting (103) the distal end (12) of the imaging device (10) into a cavity (42) in a housing (41) through an opening (43);
arranging (104) the distal end (12) of the imaging device (10) at a position relative to a reference surface (72) with predetermined optical properties in the cavity (42), said position being predetermined by a substantially tubular positioning device (50; 60), wherein the predetermined position of the distal end (12) of the imaging device (10) is within the cavity (42) and the positioning device (50) has, at its end (51) distant from the cavity (42), a mechanical stop (56) or a ball-locking device (57) for holding the proximal end (11) of an imaging device (10) that has been inserted into the test apparatus (40) or wherein the positioning device (60), at an end (62) facing the cavity (42), has a mechanical stop (66) that is embodied in such a way that the distal end (12) of an imaging device (10) that has been inserted into the test apparatus (40) lies at the predetermined position when the imaging device (10) rests against the stop (66);
illuminating (105) the reference surface (72) with illumination light having a predetermined spectrum by means of the imaging device (10);
capturing (107) light reflected by the reference surface (72) by means of the imaging device (10);
determining the functionality or another property of the optical examination system on the basis of the captured reflected light.

## Revendications

1. Dispositif de contrôle (40) pour un système d'examen optique comprenant un appareil d'imagerie (10) et une source de lumière (22) servant à l'examen optique d'un objet en lumière réémise et/ou en lumière de fluorescence, comprenant :
un boîtier (41) comportant un espace creux (42) et une ouverture (43) servant à l'insertion d'une extrémité distale (12) de l'appareil d'imagerie (10) dans l'espace creux (43) ;
une surface de référence (72) ayant des propriétés optiques prédéfinies dans l'espace creux (42), au moins soit pour la réémission de la lumière d'éclairage dirigée sur la surface de référence (72), soit pour l'émission de lumière de fluorescence ;
un appareil de positionnement (50; 60) de forme sensiblement tubulaire, destiné à maintenir l'appareil d'imagerie (10) à une position prédéfinie par l'appareil de positionnement (50; 60) de l'extrémité distale (12) de l'appareil d'imagerie (10) par rapport à la surface de référence (72),
la position prédéfinie de l'extrémité distale (12) de l'appareil d'imagerie (10) se trouvant à l'intérieur de l'espace creux (42),
l'appareil de positionnement (50) possédant, à son extrémité (41) à l'opposé de l'espace creux (42), une butée mécanique (56) ou un dispositif d'encliquetage à bille (57) servant à maintenir l'extrémité proximale (11) dans un appareil d'imagerie (10) introduit dans le dispositif de contrôle (40),
ou l'appareil de positionnement (60) possédant une butée mécanique (66) à une extrémité (62) qui fait face à l'espace creux (42), laquelle est configurée de telle sorte que l'extrémité distale (12) d'un appareil d'imagerie (10) introduit dans le dispositif de contrôle (40) se trouve à la position prédéfinie lorsque l'appareil d'imagerie (10) repose contre la butée (66).

2. Dispositif de contrôle (40) selon l'une des revendications précédentes, avec lequel l'appareil de positionnement (50 ; 60) est en outre configuré pour empêcher une rotation d'un appareil d'imagerie (10) introduit dans le dispositif de contrôle (40) autour de son axe longitudinal.

3. Dispositif de contrôle (40) selon l'une des revendications précédentes, avec lequel le dispositif de contrôle (40) est configuré de telle sorte que l'appareil de positionnement (50 ; 60) peut être séparé du boîtier (41) et de nouveau y être attaché sans utiliser d'outil.

4. Dispositif de contrôle (40) selon la revendication précédente, comprenant en outre :
des appareils (45, 53 ; 63) au niveau du boîtier (41) et au niveau de l'appareil de positionnement (50 ; 60) servant à empêcher, par complémentarité de formes, une rotation de l'appareil de positionnement (50; 60) autour de son axe longitudinal (58 ; 68).

5. Dispositif de contrôle (40) selon la revendication 3 ou 4, comprenant en outre :
un appareil d'encliquetage (46) qui est configuré pour fixer l'appareil de positionnement (50 ; 60) de manière amovible au niveau de l'ouverture (43).

6. Dispositif de contrôle (40) selon l'une des revendications précédentes, la surface de référence (72)
formant un secteur d'une surface sphérique, la position prévue pour l'extrémité distale (12) de l'appareil d'imagerie (10) se trouvant au centre de la surface sphérique ; et
formant un secteur d'une surface cylindrique circulaire, la position prévue pour l'extrémité distale (12) de l'appareil d'imagerie (10) se trouvant au niveau de l'axe de symétrie de la surface cylindrique circulaire.

7. Dispositif de contrôle (40) selon la revendication précédente, avec lequel
la surface de référence (72) forme un secteur d'une surface sphérique, deux endroits opposés (77, 78) sur le bord de la surface de référence (72) se trouvant sur une ligne droite (79) qui forme un angle dans la plage de 40 degrés à 80 degrés par rapport à l'axe longitudinal d'un appareil d'imagerie (10) maintenu par l'appareil de positionnement (50 ; 60) ; ou
la surface de référence (72) forme un secteur d'une surface sphérique, le bord de la surface de référence (72) se trouvant dans un plan (78) dont la normale forme un angle dans la plage de 10 degrés à 50 degrés par rapport à l'axe longitudinal d'un appareil d'imagerie (10) maintenu par l'appareil de positionnement (50 ; 60) ; ou
la surface de référence (72) forme un secteur d'une surface cylindrique circulaire, une ligne droite (79) à travers deux bords opposés (77, 78) de la surface de référence (72) formant un angle compris entre 40 degrés et 80 degrés par rapport à l'axe longitudinal d'un appareil d'imagerie (10) maintenu par l'appareil de positionnement (50 ; 60).

8. Dispositif de contrôle (40) selon l'une des revendications précédentes, le dispositif de contrôle étant démontable et stérilisable.

9. Dispositif de contrôle (40) selon l'une des revendications précédentes, la surface de référence (72) étant blanche ou comportant une zone blanche.

10. Dispositif de contrôle (40) selon l'une des revendications précédentes, avec lequel la surface de référence (72) est formée par du polytétrafluoro-éthylène ou du silicone.

11. Procédé de contrôle d'un système d'examen optique comprenant un appareil d'imagerie (10) et une source de lumière (22) servant à l'examen optique d'un objet en lumière réémise et en lumière de fluorescence, comprenant les étapes suivantes :
introduction (103) de l'extrémité distale (12) de l'appareil d'imagerie (10) à travers une ouverture (43) dans un espace creux (42) dans un boîtier (41) ;
arrangement (104) de l'extrémité distale (12) de l'appareil d'imagerie (10) à une position prédéfinie par un appareil de positionnement (50 ; 60) de forme sensiblement tubulaire par rapport à une surface de référence (72) ayant des propriétés optiques prédéfinies dans l'espace creux (42), la position prédéfinie de l'extrémité distale (12) de l'appareil d'imagerie (10) se trouvant à l'intérieur de l'espace creux (42) et l'appareil de positionnement (50) possédant, à son extrémité (41) à l'opposé de l'espace creux (42), une butée mécanique (56) ou un dispositif d'encliquetage à bille (57) servant à maintenir l'extrémité proximale (11) dans un appareil d'imagerie (10) introduit dans le dispositif de contrôle (40), ou l'appareil de positionnement (60) possédant une butée mécanique (66) à une extrémité (62) qui fait face à l'espace creux (42), laquelle est configurée de telle sorte que l'extrémité distale (12) d'un appareil d'imagerie (10) introduit dans le dispositif de contrôle (40) se trouve à la position prédéfinie lorsque l'appareil d'imagerie (10) repose contre la butée (66) ;
éclairage (105) de la surface de référence (72) avec de la lumière d'éclairage ayant un spectre prédéfini au moyen de l'appareil d'imagerie (10) ;
détection (107) de la lumière réémise par la surface de référence (72) au moyen de l'appareil d'imagerie (10) ;
détermination de l'aptitude fonctionnelle ou d'une autre propriété du système d'examen optique à l'aide de la lumière réémise détectée.
